# EUROPEAN PATENT SPECIFICATION

(11) **EP 1 968 571 B1**
(45) Date of publication and mention of the grant of the patent: **07.12.2011**
(21) Application number: 06845823.1
(22) Date of filing: 19.12.2006
(51) Int. Cl.: A61K 31/353, A61K 31/357, A61P 25/02, A61P 25/08, A61P 25/14, A61P 25/16

(54) **USE OF BENZO-FUSED HETEROCYCLE SULFAMIDE DERIVATIVES AS NEUROPROTECTIVE AGENTS**
VERWENDUNG VON BENZOKONDENSIERTEN HETEROZYKLISCHEN SULFAMID-DERIVATEN ALS NEUROPROTEKTIVE MITTEL
UTILISATION DE DERIVES HETEROCYCLIQUES BENZO-CONDENSES DE SULFAMIDE EN TANT QU'AGENTS PROTECTEURS DU SYSTEME NERVEUX

(30) Priority: 19.12.2005 US 751494 P; 18.12.2006 US 612146
(43) Date of publication of application: 17.09.2008
(73) Proprietor: Janssen Pharmaceutica N.V., 2340 Beerse (BE)
(72) Inventor: SMITH-SWINTOSKY, Virginia, L., Hatfield, Pennsylvania 19440 (US); REITZ, Allen, B., Lansdale, Pennsylvania 19446 (US)
(74) Representative: Warner, James Alexander
(86) International application number: PCT/US2006/048451
(87) International publication number: WO 2007/075698

(56) References cited:
- WO-A-2004/014352
- WO-A-2006/007436
- US-A1- 2004 253 223
- US-B1- 6 949 518
- MARYANOFF B E ET AL: "Comparison of sulfamate and sulfamide groups for the inhibition of carbonic anhydrase-II by using topiramate as a structural platform" JOURNAL OF MEDICINAL CHEMISTRY 24 MAR 2005 UNITED STATES, vol. 48, no. 6, 24 March 2005 (2005-03-24), pages 1941-1947, XP002431411 ISSN: 0022-2623
- TENOVUO O: "Central acetylcholinesterase inhibitors in the treatment of chronic traumatic brain injury - Clinical experience in 111 patients" PROGRESS IN NEURO-PSYCHOPHARMACOLOGY AND BIOLOGICAL PSYCHIATRY 2005 UNITED STATES, vol. 29, no. 1, January 2005 (2005-01), pages 61-67, XP002431412 ISSN: 0278-5846
- SCOZZAFAVA A ET AL: "Modulation of carbonic anhydrase activity and its applications in therapy" EXPERT OPINION ON THERAPEUTIC PATENTS 2004 UNITED KINGDOM, vol. 14, no. 5, 2004, pages 667-702, XP002431413 ISSN: 1354-3776
- SHANK R P ET AL: "Examination of two independent kinetic assays for determining the inhibition of carbonic anhydrases I and II: Structure-activity comparison of sulfamates and sulfamides" CHEMICAL BIOLOGY AND DRUG DESIGN 2006 UNITED KINGDOM, vol. 68, no. 2, 2006, pages 113-119, XP002431414 ISSN: 1747-0277
- KLINGER A L ET AL: "Inhibition of carbonic anhydrase-II by sulfamate and sulfamide groups: An investigation involving direct thermodynamic binding measurements" JOURNAL OF MEDICINAL CHEMISTRY 15 JUN 2006 UNITED STATES, vol. 49, no. 12, 15 June 2006 (2006-06-15), pages 3496-3500, XP002431415 ISSN: 0022-2623

## Description

### FIELD OF THE INVENTION

The present invention is directed to the use of benzo-fused heterocycle sulfamide derivatives as neuroprotective agents. The present invention is further directed to the use of benzo-fuzed heterocycle sulfamide derivatives for the treatment of acute and / or chronic neurodegenerative disorders, more particularly for the treatment of acute or chronic neurodegenerative disorders characterized by neuron damage or death.

### BACKGROUND OF THE INVENTION

Neurodegenerative conditions afflict a wide variety of individuals, both in the U.S. and abroad. For example, many individuals suffer from neurodegenerative diseases. These diseases include a range of seriously debilitating conditions, such as Parkinson's disease, amyotrophic lateral sclerosis (ALS, "Lou Gehrig's disease"), multiple sclerosis, Huntington's disease, Alzheimer's disease, diabetic retinopathy, multi-infarct dementia, macular degeneration.

Increased longevity in humans has led to an increased awareness of the prevalence of neurodegenerative disease. The relatively high incidence - 2 - of these diseases (reports range from between 2-15% of the population over 70 years of age) poses significant medical, social, and financial burdens on sufferers, care-givers, and the general community. Following onset, these diseases can lead to death very quickly, or alternatively, they can be slowly progressive over a period of years, often culminating in the sufferer requiring dedicated institutionalized care.

As the population ages, the frequency with which patients are diagnosed with neurodegenerative diseases, especially those which affect mental faculties such as Alzheimer's, is growing dramatically. The number of individuals having Alzheimer's disease is growing exponentially and it is estimated that today there may be as many as 24 million individuals worldwide afflicted with this condition.

Alzheimer's Disease (AD) is caused by a degenerative process in the patient which is characterized by progressive loss of cells from the basal forebrain, cerebral cortex and other brain areas. Acetylcholine transmitting neurons and their target nerves are particularly affected. Senile plaques and neurofibrillary tangles are present. Pick's disease has a similar clinical- picture to Alzheimer's disease but a somewhat slower clinical course and circumscribed atrophy, mainly affecting the frontal and temporal lobes . One animal model for Alzheimer's disease and other dementias displays hereditary tendency toward the formation of such plaques. It is thought that if a drug has an effect in the model, lit also may be beneficial in at least some forms of Alzheimer's and Pick's diseases. At present there are palliative treatments but no means to restore function in Alzheimer's patients.

Parkinson's disease (PD), is a disorder of middle or late life, with very gradual progression and a prolonged course. HARRISON'S PRINCIPLES OF INTERNAL MEDICINE, Vol. 2; 23d ed., Ed by lsselbacher, Braunwald, Wilson, Martin, Fauci and Kasper, McGraw-Hill Inc. , New York City, 1994, pg. 2275. The most regularly observed changes in patients with Parkinson's disease have been in the aggregates of melanin- containing nerve cells in the brainstem (substantia nigra, locus 20 coeruleus), where there are varying degrees of nerve cell loss with reactive gliosis (most pronounced in the substantia nigra) along with distinctive eosinophilic intracytoplasmic inclusions. In its fully developed form, PD is easily recognized in patients, where stooped posture, stiffness and slowness of movement, fixity of facial expression, rhythmic tremor of the limbs, which subsides on active willed movement or complete relaxation, are common features. Generally, accompanying the other characteristics of the fully developed disorder is the festinating gait, whereby the patient, progresses or walks with quick shuffling steps at an accelerating pace as if to catch up with the body's center of gravity.

The treatment of Parkinson's disease pharmacologically with levodopa combined with stereotactic surgery has only represented a partial cure, at best. Underlying much of the treatment difficulty is directed to the fact that none of these therapeutic measures has an effect on the underlying disease process, which consists of neuronal degeneration. Ultimately, a point seems to be reached where pharmacology can no longer compensate for the loss of basal ganglia dopamine.

Other neurodegenerative conditions afflicting humans result from or are otherwise caused, at least in part, by stroke or other trauma or injury. According to one source, as many as 700,000 new cases of stroke occur each year. In the U.S., a stroke occurs every minute. The majority of stroke patients sustain permanent disability, and stroke is the leading cause of neurological disability in adults, affecting 3-4 million U.S. citizens.

US6949518 discloses the use of topiramate in treating optic nerve degeneration.

There remains a need to provide an effective treatment for acute and chronic neurodegenerative disorders. Further, there remains a need for agents which are neuroprotective and are therefore useful for the prevention of neuron death and / or damage.

### SUMMARY OF THE INVENTION

The present invention is directed to a method for neuroprotection comprising administering to a subject in need thereof a therapeutically effective amount of a compound of formula (I) 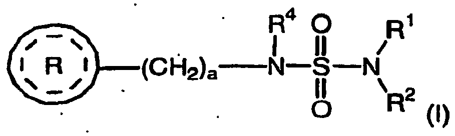 wherein
R¹ and R² are each independently selected from the group consisting of hydrogen and lower alkyl;
R⁴ is selected from the group consisting of hydrogen and lower alkyl;
a is an integer from 1 to 2;
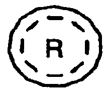 is selected from the group consisting of 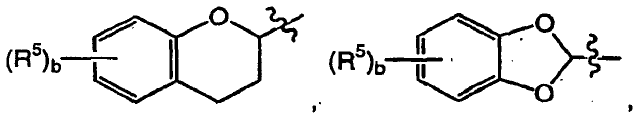 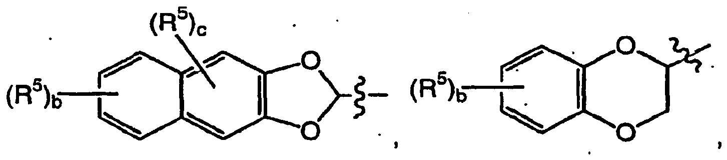 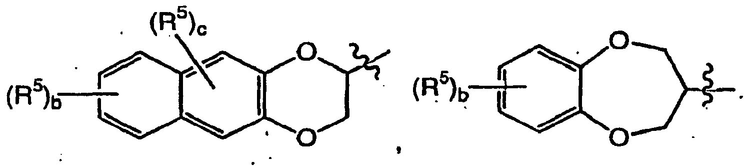 and 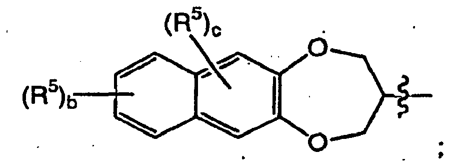 wherein b is an integer from 0 to 4; and wherein c is an integer from 0 to 2;
each R⁵ is independently selected from the group consisting of halogen, lower alkyl and nitro;
provided that when 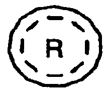 is 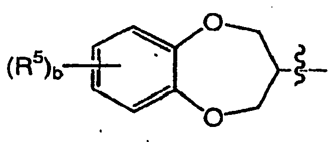 or 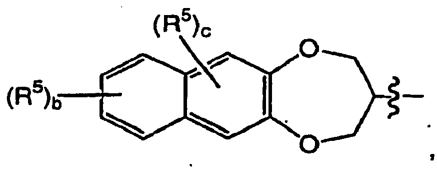 then a is 1;
or a pharmaceutically acceptable salt thereof.

The present invention is further directed to a method for neuroprotection comprising administering to a subject in need thereof a therapeutically effective amount of compound of formula (II) 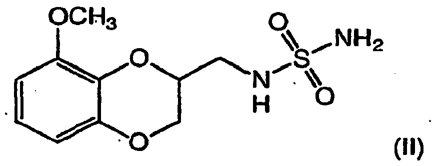 or a pharmaceutically acceptable salt thereof.

Exemplifying the invention is a method for neuroprotection comprising administering to a subject in need thereof a therapeutically effective amount of any of the compounds described above.

In an example, the invention relates to a method for the treatment of acute neurodegenerative disorders comprising administering to a subject in need thereof a therapeutically effective amount of any of the compounds described above. In another example, the invention relates to a method for the treatment of chronic neurodegenerative disorders.

Further exemplifying the invention is a method for preventing neuron death or damage following an insult or injury to the brain, central nervous system or peripheral nervous system.

### DETAILED DESCRIPTION OF THE INVENTION

The present invention relates to a method for neuroprotection comprising administering to a subject in need thereof a therapeutically effective amount of a compound of formula (I) 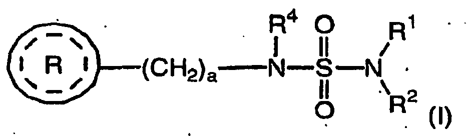 or a pharmaceutically acceptable salt thereof, wherein 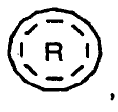 a, R1, R² and R⁴ are as herein defined. The present invention is further directed to a method for the treatment of acute or chronic neurodegenerative disorders. The present invention is further directed to a method for the preventing neuron death or damage following injury.

As used herein, the term "**neuroprotection**" shall mean the protecting neurons in the brain, central nervous system or peripheral nervous system (preferably in the brain or spinal cord) from death and / or damage. Preferably, the neurons are protected from death or damage caused by oxidative stress, for example oxygen radicals.

"**Acute neurodegenerative disorders**" included within the methods of the present invention include, but are not limited, to various types of acute neurodegenerative disorders associated with neuron death or damage including cerebrovascular insufficiency, focal brain trauma, diffuse brain damage, and spinal cord injury, that is, cerebral ischemia or infarction including embolic occlusion and thrombotic occlusion, reperfusion following acute ischemia, perinatal hypoxic-ischemic injury, cardiac arrest, as well as intracranial hemorrhage of any type (including epidural, subdural, subarachnoid and intracerebral), and intracranial and intravertebral lesions (including contusion, penetration, shear, compression and laceration), and whiplash shaken infant syndrome. Preferably, the acute neurodegenerative disorder is a result of stroke, acute ischemic injury, head injury or spinal injury.

"**Chronic neurodegenerative disorders**" included within the methods of the present invention included, Alzeimer's disease, Pick's disease, diffuse Lewy body disease, progressive supranuclear palsy (Steel-Richardson syndrome), multisystem degeneration (Shy-Drager syndrome), chronic epileptic conditions associated with neurodegeneration, motor neuron diseases including amyotrophic lateral sclerosis, degenerative ataxias, cortical basal degeneration, ALS-Parkinson's-Dementia complex of Guam, subacute sclerosing panencephalitis, Huntington's disease, Parkinson's disease, synucleinopathies (including multiple system atrophy), primary progressive aphasia, striatonigral degeneration, Machado-Joseph disease / spinocerebellar ataxia type 3 and olivopontocerebellar degenerations, Gilles De La Tourette's disease, bulbar and pseudobulbar palsy, spinal and spinobulbar muscular atrophy (Kennedy's disease), multiple sclerosis, primary lateral sclerosis, familial spastic paraplegia, Werdnig-Hoffmann disease, Kugelberg-Welander disease, Tay-Sach's disease, Sandhoff disease, familial spastic disease, Wohlfart-Kugelberg-Welander disease, spastic paraparesis, progressive multifocal leukoencephalopathy, familial dysautonomia (Riley-Day syndrome), and prion diseases (including Creutzfeldt-Jakob, Gerstmann-Sträussler-Scheinker disease, Kuru and fatal familial insomnia). Preferably, the chronic neurodegenerative disorder is selected from Alzheimer's disease, Parkinson's disease, multiple sclerosis or.cerebral palsy,

Other disorders which manifest neuron death or damage and as such are intended to be included within the methods of the present invention include dementias, regardless of underlying etiology, including age-related dementia and other dementias and conditions with memory loss including dementia associated with Alzheimer's disease, vascular dementia, diffuse white matter disease (Binswanger's disease), dementia of endocrine or metabolic origin, dementia of head trauma and diffuse_brain damage, dementia pugilistica and frontal lobe dementia.

Also included within the present invention are methods of neuroprotection (i.e. methods for the prevention of neuron death and / or damage) following injury to the brain, central nervous system or peripheral nervous system, wherein the injury resulting from chemical, toxic, infectious, radiation and / or traumatic injury. Preferably, the methods of the present invention are directed to preventing neuron death or damage following brain, head and / or spinal cord trauma or injury, regardless of cause.

The term "**subject**" as used herein, refers to an animal, preferably a mammal, most preferably a human, who has been the object of treatment, observation or experiment.

The term "**therapeutically effective amount**" as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

In an embodiment of the present invention R¹ is selected from the group consisting of hydrogen and methyl. In another embodiment of the present invention R² is selected from the group consisting of hydrogen and methyl. In yet another embodiment of the present invention R¹ and R² are each hydrogen or R¹ and R² are each methyl.

In an embodiment of the present invention -(CH₂)ₐ- is selected from the group consisting of -CH₂- and -CH₂-CH₂-. In another embodiment of the present invention -(CH₂)ₐ- is -CH₂-.

In an embodiment of the present R⁴ is selected from the group consisting of hydrogen and methyl, preferably, R⁴ is hydrogen.

In an embodiment of the present invention a is 1.

In an embodiment of the present invention b is an integer from 0 to 2. In another embodiment of the present invention c is an integer from 0 to 2. In another embodiment of the present invention b is an integer from 0 to 1. In another embodiment of the present invention c is an integer from 0 to 1. In yet another embodiment of the present invention the sum of b and c is an integer form 0 to 2, preferably an integer form 0 to 1. In yet another embodiment of the present invention b is an integer from 0 to 2 and c is 0.

In an embodiment of the present invention, 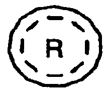 is selected from the group consisting of 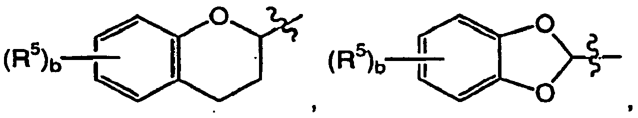 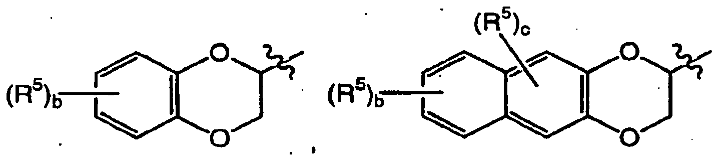 and 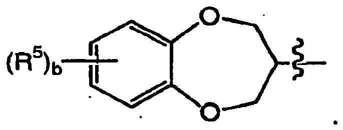 In another embodiment of the present invention, 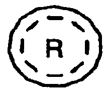 is selected from the group consisting of

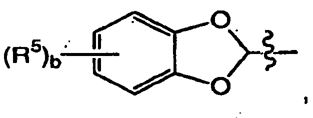

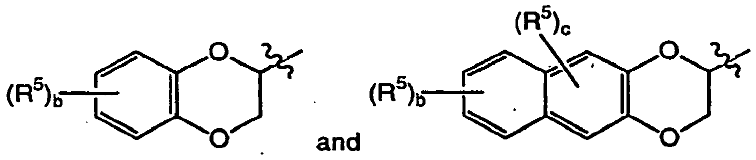

In an embodiment of the present invention, 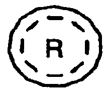 is selected from the group consisting of 2-(2,3-dihydro-benzo[1,4]dioxinyl), 2-(benzo[1,3]dioxolyl), 3-(3,4-dihydro-benzo[1,4]dioxepinyl), 2-(6-chloro-2,3-dihydro-benzo[1,4]dioxinyl), 2-(6-fluoro-2,3-dihydro-benzo[1,4]dioxinyl), 2-(chromanyl), 2-(5-fluoro-2,3-dihydro-benzo[1,4]dioxinyl), 2-(7-chloro-2,3-dihydro-benzo[1,4]dioxinyl), 2-(6-chloro-benzo[1,3]dioxolyl), 2-(7-nitro-2,3-dihydro-benzo[1,4]dioxinyl), 2-(7-methyl-2,3-dihydro-benzo[1,4]dioxinyl), 2-(5-chloro-2,3-dihydro-benzo[1,4]dioxinyl), 2-(6-bromo-2,3-dihydro-benzo[1,4]dioxinyl), 2-(6,7-dichloro-2,3-dihydro-benzo[1,4]dioxinyl), 2-(8-chloro-2,3-dihydro-benzo[1,4]dioxinyl); 2-(2,3-dihydro-naphtho[2,3-b][1,4]dioxinyl) and 2-(4-methyl-benzo[1,3]dioxolyl).

In another embodiment of the present invention, 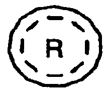 is selected from the group consisting 2-(benzo[1,3]dioxolyl), 2-(2,3-dihydro-benzo[1,4]dioxinyl), 2-(6-chloro-2,3-dihydro-benzo[1,4]dioxinyl), 2-(7-chloro-2,3-dihydro-benzo[1,4]dioxinyl), 2-(7-methyl-2,3-dihydro-benzo[1,4]dioxinyl), 2-(6-bromo-2,3-dihydro-benzo[1,4]dioxinyl) and 2-(6,7-dichloro-2,3-dihydro-benzo[1,4]dioxinyl). In another embodiment of the present invention, 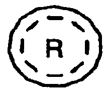 is selected from the group consisting of 2-(2,3-dihydro-benzo[1,4]dioxinyl), 2-(7-methyl-2,3-dihydro-benzo[1,4]dioxinyl) and 2-(6-bromo-2,3-dihydro-benzo[1,4]dioxinyl).

In an embodiment of the present invention R⁵ is selected from the group consisting of halogen and lower alkyl. In another embodiment of the present invention R⁵ is selected from chloro, fluoro, bromo and methyl.

In an embodiment of the present invention, the stereo-center on the compound of formula (I) is in the S-configuration. In another embodiment of the present invention, the stereo-center on the compound of formula (I) is in the R-configuration.

In an embodiment of the present invention the compound of formula (I) is present as an enantiomerically enriched mixture, wherein the % enantiomeric enrichment (% ee) is greater than about 75%, preferably greater than about 90%, more preferably greater than about 95%, most preferably greater than about 98%.

Additional embodiments of the present invention, include those wherein the substituents selected for one or more of the variables defined herein (i.e. R¹, R², R³, R⁴, X-Y and A) are independently selected to be any individual substituent or any subset of substituents selected from the complete list as defined herein.

Representative compounds of the present invention, are as listed in Tables 1 below. Additional compounds of the present invention are as listed in Table 3. In Tables 1 and 2 below, the column headed "stereo" defines the stereo-configuration at the carbon atom of the heterocycle attached at the starred bond. Where no designation is listed, the compound was prepared as a mixture of stereo-configurations. Where an "R" or "S" designation is listed, the stereo-configuration was based on the enantiomerically enriched starting material.

**Table 1: Representative Compounds of Formula (I)**

| | | | | | | |
|---|---|---|---|---|---|---|
| 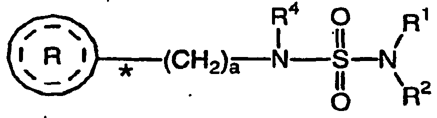 | | | | | | |

| **ID No.** | 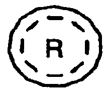 | **Stereo** | **(CH₂)ₐ** | **NR⁴** | **R¹** | **R²** |
|---|---|---|---|---|---|---|
| 1 | 2-(2,3-dihydro-benzo[1,4]dioxinyl) | | CH₂ | NH | H | H |
| 2 | 2-(benzo[1,3]dioxolyl) | | CH₂ | NH | H | H |
| 3 | 3-(3,4-dihydro-2H-benzo[1,4]dioxepinyl) | | CH₂ | NH | H | H |
| 4 | 2-(2,3-dihydro-benzo[1,4]dioxinyl). | S | CH₂ | NH | H | H |
| 5 | 2-(2,3-dihydro-benzo[1,4]dioxinyl) | R | CH₂ | NH | H | H |
| 6 | 2-(2,3-dihydro-benzo[1,4]dioxinyl) | | CH₂ | NH | methyl | methyl |
| 7 | 2-(2,3-dihydro-benzo[1,4]dioxinyl) | | CH₂ | N(CH₃) | H | H |
| 8 | 2-(6-chloro-2,3-dihydro-benzo[1,4]dioxinyl) | S | CH₂ | NH | H | H |
| 9 | 2-(6-fluoro-2,3-dihydro-benzo[1,4]dioxinyl) | S | CH₂ | NH | H | H |
| 10 | 2-(chromanyl) | | CH₂ | NH | H | H |
| 13 | 2-(5-fluoro-2,3-dihydro-benzo[1,4]dioxinyl) | S | CH₂ | NH | H | H |
| 14 | 2-(7-chloro-2,3-dihydro-benzo[1,4]dioxinyl) | S | CH₂ | NH | H | H |
| 15 | 2-(6-chloro-benzo[1,3]dioxolyl) | | CH₂ | NH | H | H |
| 16 | 2-(2,3-dihydro-benzo[1,4]dioxinyl) | | CH₂CH₂ | NH | H | H |
| 18 | 2-(7-nitro-2,3-dihydro-benzo[1,4]dioxinyl) | S | CH₂ | NH | H | H |
| 19 | 2-(7-methyl-2,3-dihydro-benzo[1,4]dioxinyl) | S | CH₂ | NH | H | H |
| 20 | 2-(5-chloro-2,3-dihydro-benzo[1,4]dioxinyl) | S | CH₂ | NH | H | H |
| 22 | 2-(8-methoxy-2,3-dihydro-benzo[1,4]dioxinyl) | S | CH₂ | NH | H | H |
| 24 | 2-(6-bromo-2,3-dihydro-benzo[1,4]dioxinyl) | S | CH₂ | NH | H | H |
| 29 | 2-(6,7-dichloro-2,3-dihydro-benzo[1,4]dioxinyl) | S | CH₂ | NH | H | H |
| 30 | 2-(8-chloro-2,3-dihydro-benzo[1,4]dioxinyl) | S | CH₂ | NH | H | H |
| 33 | 2-(2,3-dihydro-naphtho[2,3-b][1,4]dioxinyl) | S | CH₂ | NH | H | H |
| 35 | 2-(4-methyl-benzo[1,3]dioxolyl) | | CH₂ | NH | H | H |

**Table 2: Additional Compounds of the Present Invention**

| | | | | | | |
|---|---|---|---|---|---|---|
| 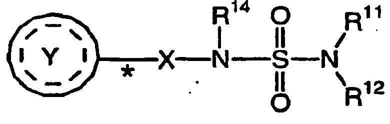 | | | | | | |

| **ID No.** | 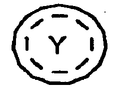 | **Stereo** | **X** | **NR¹⁴** | **R¹¹** | **R¹²** |
|---|---|---|---|---|---|---|
| 23 | 2-(5-methoxy-2,3-dihydro-benzo[1,4]dioxinyl) | S | CH₂ | NH | H | H |
| 26 | 2-(6-methylcarbonyl-2,3-dihydro-benzo[1,4]dioxinyl) | S | CH₂ | NH | H | H |
| 32 | 2-(6-methoxycarbonyl-2,3-dihydro-benzo[1,4]dioxinyl) | S | CH₂ | NH | H | H |
| 34 | 2-(6-hydroxymethyl-2,3-dihydro-benzo[1,4]dioxinyl) | S | CH₂ | NH | H | H |
| 36 | 2-(7-amino-2,3-dihydro-benzo[1,4]dioxinyl) | S | CH₂ | NH | H | H |

As used herein, unless otherwise noted, "**halogen**" shall mean chlorine, bromine, fluorine and iodine.

As used herein, unless otherwise noted, the term "**alkyl**" whether used alone or as part of a substituent group, includes straight and branched chains. For example, alkyl radicals include methyl, ethyl, propyl, isopropyl, butyl, isobutyl, sec-butyl, t-butyl and pentyl. Unless otherwise noted, "lower" when used with alkyl means a carbon chain composition of 1-4 carbon atoms.

As used herein, unless otherwise noted, "**alkoxy**" shall denote an oxygen ether radical of the above described straight or branched chain alkyl groups. For example, methoxy, ethoxy, n-propoxy, sec-butoxy, t-butoxy and n-hexyloxy.

As used herein, the notation "*" shall denote the presence of a stereogenic center.

When a particular group is "**substituted**" (e.g., alkyl, aryl, etc.), that group may have one or more substituents, preferably from one to five substituents, more preferably from one to three substituents, most preferably from one to two substituents, independently selected from the list of substituents.

With reference to substituents, the term "**independently**" means that when more than one of such substituents is possible, such substituents may be the same or different from each other.

Under standard nomenclature used throughout this disclosure, the terminal portion of the designated side chain is described first, followed by the adjacent functionality toward the point of attachment. Thus, for example, a "**phenyl-alkyl-amino-carbonyl-alkyl**" substituent refers to a group of the formula

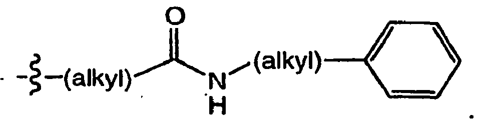

Abbreviations used in the specification, particularly the Schemes and Examples, are as follows:
- DCC =: Dicyclohexyl Carbodiimide
- DCE =: Dichloroethane
- DCM =: Dichloromethane
- DIPEA or DIEA =: Diisopropylethylamine
- DMF =: N,N-Dimethylformamide
- DMSO =: Dimethylsulfoxide
- EDC =: Ethylcarbodiimide
- Et₃N or TEA =: Triethylamine
- Et₂O =: Diethyl ether
- EA or EtOAc =: Ethyl acetate
- EtOH =: Ethanol
- IPA =: 2-propanol
- Hept =: Heptane
- HOBT =: 1-Hydroxybenzotriazole
- HPLC =: High Pressure Liquid Chromatography
- LAH =: Lithium Aluminum Hydride
- M or MeOH =: Methanol
- NMR =: Nuclear Magnetic Resonance
- Pd-C =: Palladium on Carbon Catalyst
- RP HPLC =: Reverse Phase High Pressure Liquid Chromatography
- RT or rt =: Room temperature
- TEA =: Triethylamine
- TFA =: Trifluoroacetic Acid
- THF =: Tetrahydrofuran
- TLC =: Thin Layer Chromatography

Where the compounds according to this invention have at least one chiral center, they may accordingly exist as enantiomers. Where the compounds possess two or more chiral centers, they may additionally exist as diastereomers. It is to be understood that all such isomers and mixtures thereof are encompassed within the scope of the present invention. Furthermore, some of the crystalline forms for the compounds may exist as polymorphs and as such are intended to be included in the present invention. In addition, some of the compounds may form solvates with water (i.e., hydrates) or common organic solvents, and such solvates are also intended to be encompassed within the scope of this invention.

For use in medicine, the salts of the compounds of this invention refer to non-toxic "**pharmaceutically acceptable salts.**" Other salts may, however, be useful in the preparation of compounds according to this invention or of their pharmaceutically acceptable salts. Suitable pharmaceutically acceptable salts of the compounds include acid addition salts which may, for example, be formed by mixing a solution of the compound with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compounds of the invention carry an acidic moiety, suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts. Thus, representative pharmaceutically acceptable salts include the following:
acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, oleate, pamoate (embonate), palmitate, pantothenate, phosphate/diphosphate, polygalacturonate, salicylate, stearate, sulfate, subacetate, succinate, tannate, tartrate, teoclate, tosylate, triethiodide and valerate.

Representative acids and bases which may be used in the preparation of pharmaceutically acceptable salts include the following:
acids including acetic acid, 2,2-dichloroactic acid, acylated amino acids, adipic acid, alginic acid, ascorbic acid, L-aspartic acid, benzenesulfonic acid, benzoic acid, 4-acetamidobenzoic acid, (+)-camphoric acid, camphorsulfonic acid, (+)-(I S)-camphor-1 0-sulfonic acid, capric acid, caproic acid, caprylic acid, cinnamic acid, citric acid, cyclamic acid, dodecylsulfuric acid, ethane-1,2-disulfonic acid, ethanesulfonic acid, 2-hydrocy-ethahesulfonic acid, formic acid, fumaric acid, galactaric acid, gentisic acid, glucoheptonic acid, D-gluconic acid, D-glucoronic acid, L-glutamic acid, α-oxo-glutaric acid, glycolic acid, hipuric acid, hydrobromic acid, hydrochloric acid, (+)-L-lactic acid, (±)-DL-Lactic acid, lactobionic acid, maleic acid, (-)-L-malic acid, malonic acid, (±)-DL-mandelic acid, methanesulfonic acid, naphthalene-2-sulfonic acid, naphthalene-1,5-disulfonic acid, 1-hydroxy-2-naphthoic acid, nicotine acid, nitric acid, oleic acid, orotic acid, oxalic acid, palmitric acid, pamoic acid, phosphoric acid, L-pyroglutamic acid, salicylic acid, 4-amino-salicylic acid, sebaic acid, stearic acid, succinic acid, sulfuric acid, tannic acid, (+)-L-tartaric acid, thiocyanic acid, p-toluenesulfonic acid and undecylenic acid; and
bases including ammonia, L-arginine, benethamine, benzathine, calcium hydroxide, choline, deanol, diethanolamine, diethylamine, 2-(diethylamino)-ethanol, ethanolamine, ethylenediamine, N-methyl-glucamine, hydrabamine, 1H-imidazole, L-lysine, magnesium hydroxide, 4-(2-hydroxyethyl)-morpholine, piperazine, potassium hydroxide, 1-(2-hydroxyethyl)-pyrrolidine, secondary amine, sodium hydroxide, triethanolamine, tromethamine and zinc hydroxide.

Compounds of formula (I) may be prepared according to the process outlined in Scheme 1.

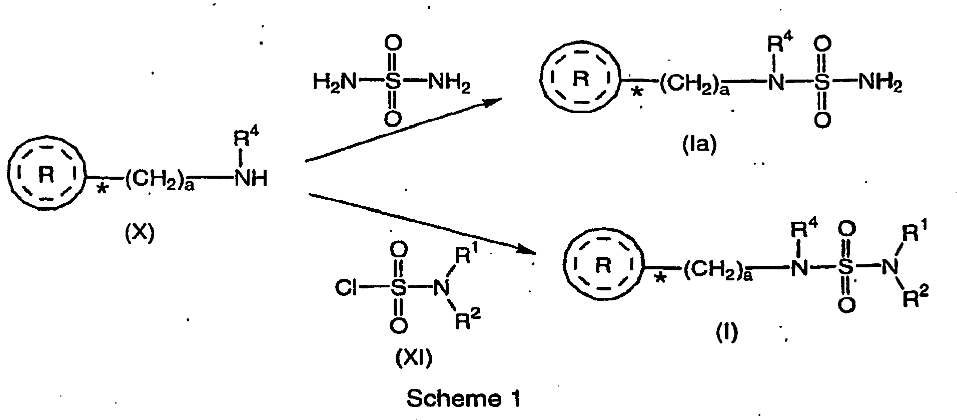

Accordingly, a suitably substituted compound of formula (X), a known compound or compound prepared by known methods, is reacted with sulfamide, a known compound, preferably wherein the sulfamide is present in an amount in the range of about 2 to about 5 equivalents, in an organic solvent such as THF, dioxane, preferably at an elevated temperature in the range of about 50°C to about 100°C, more preferably at about reflux temperature, to yield the corresponding compound of formula (Ia).

Alternatively, a suitably substituted compound of formula (X), a known compound or compound prepared by known methods, is reacted with a suitably substituted compound of formula (XI), a known compound or compound prepared by known methods, in the presence of a base such as TEA, DIPEA, pyridine, in an organic solvent such as DMF, DMSO, to yield the corresponding compound of formula (I).

Compounds of formula (X) wherein 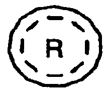 is 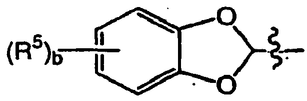 may be prepared according to the process outlined in Scheme 2.

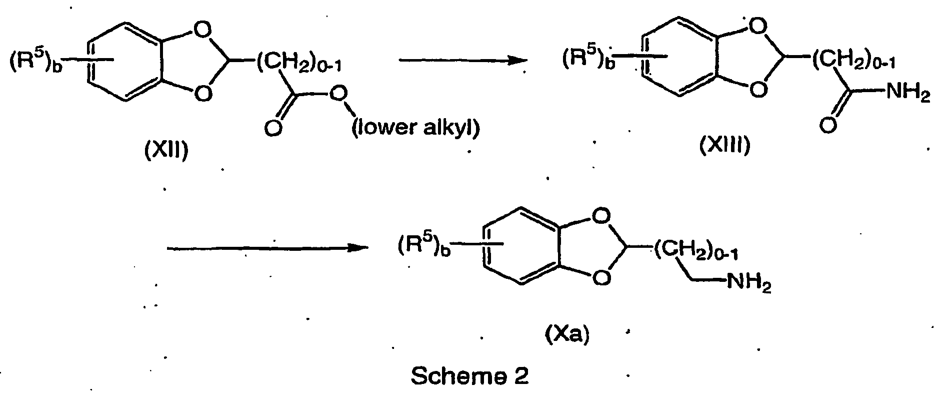

Accordingly, a suitably substituted compound of formula (XII), a known compound or compound prepared by known method (for example as described in Scheme 3 above) is reacted with NH₄OH, a known compound, optionally in an organic solvent such as acetonitrile, to yield the corresponding compound of formula (XIII).

The compound of formula (XIII) is reacted with a suitably selected reducing agent, such as LAH, in an organic solvent such as THF, diethyl ether, to yield the corresponding compound of formula (Xa).

Compounds of formula (X) wherein 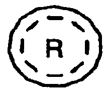 is selected from 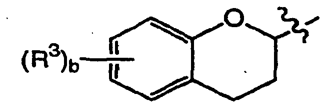 may be prepared according to the process outlined in Scheme 3.

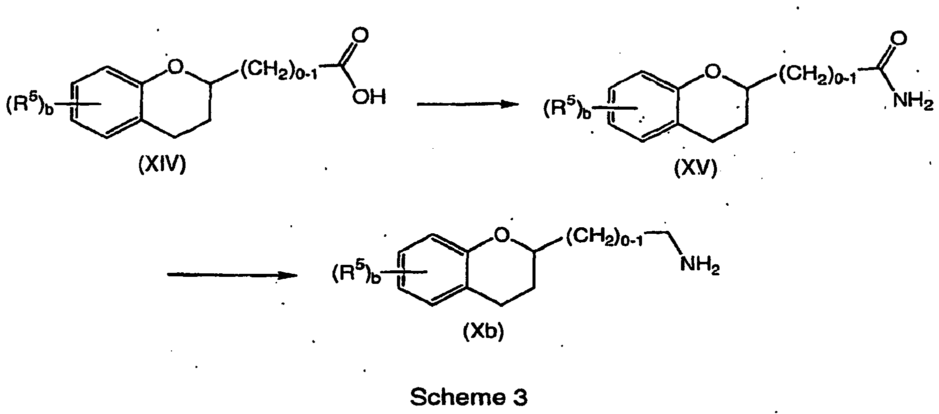

Accordingly, a suitably substituted compound of formula (XIV), a known compound or compound prepared by known methods, is reacted with NH₄OH, in the presence of a coupling agent such as DCC, optionally in an organic solvent such as acetonitrile, to yield the corresponding compound of formula (XV).

The compound of formula (XV) is reacted with a suitably selected reducing agent, such as LAH, in an organic solvent such as THF, diethyl ether, to yield the corresponding compound of formula (Xb).

Compounds of formula (X) wherein 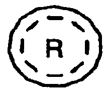 is selected from 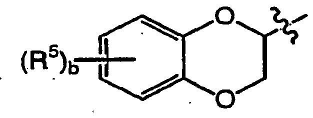 and wherein a is 2, may be prepared according to the process outlined in Scheme 4.

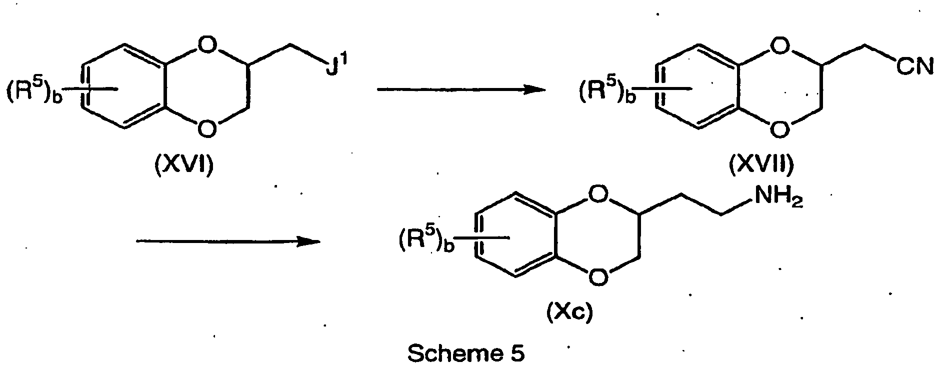

Accordingly, a suitably substituted compound of formula (XVI) wherein J¹ is a suitable leaving group such as Br, CI, I, tosyl, mesyl, triflyl, a known compound or compound prepared by known methods (for example, by activating the corresponding compound wherein J¹ is OH), is reacted with a cyanide such as potassium cyanide, sodium cyanide, in an organic solvent such as DMSO, DMF, THF, to yield the corresponding compound of formula (XVII).

The compound of formula (XVII) is reduced according to known methods, for example by reacting with a suitable reducing agent such as LAH, borane, to yield the corresponding compound of formula (Xc).

Compounds of formula (X) wherein 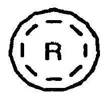 is selected from 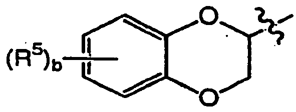 and wherein a is 1, may be prepared according to the process outlined in Scheme 5.

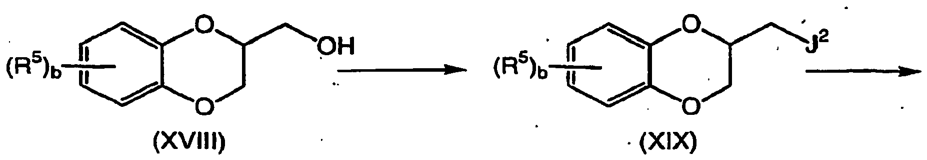

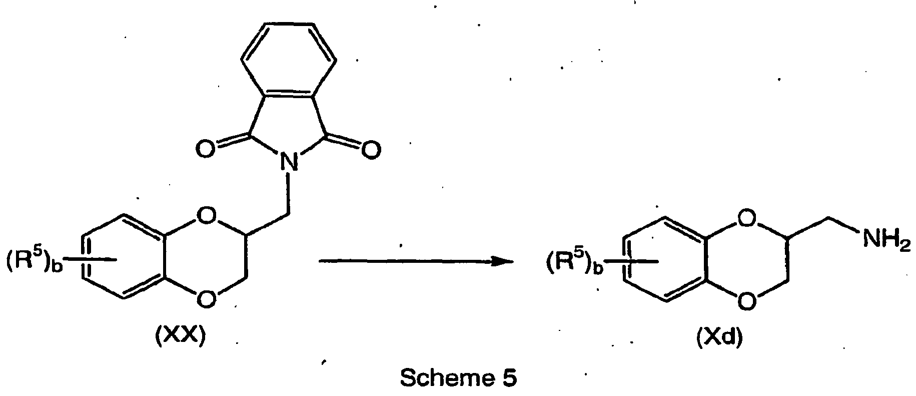

Accordingly, a suitably substituted compound of formula (XVIII), a known compound or compound prepared by known methods is activated, according to known method, to yield the corresponding compound of formula (XIX), wherein J² is a suitable leaving group, such tosylate, Cl, Br, I, mesylate and triflate.

The compound of formula (XIX) is reacted with a phthalimide salt such as potassium phthlimide, sodium phthalimide, in an organic solvent such as DMF, DMSO, acetonitrile, preferably, at an elevated temperature in the range of from 50°C to about 200°C, more preferably, at about reflux temperature, to yield the corresponding compound of formula (XX).

The compound of formula (XX) is reacted with N₂H₄, a known compound, in an organic solvent such as ethanol, methanol, preferably, at an elevated temperature in the range of from about 50°C to about 100°C, more preferably, at about reflux temperature, to yield the corresponding compound of formula (Xd).

One skilled in the art will recognize that compounds of formula (X) wherein 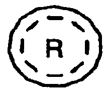 is selected from 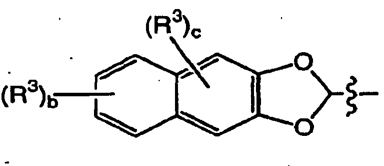 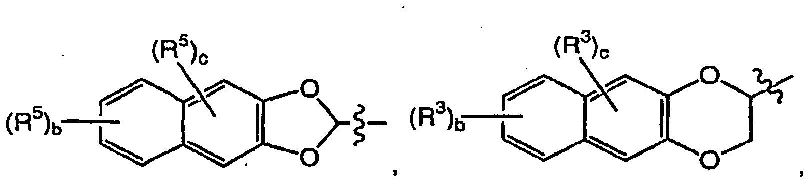 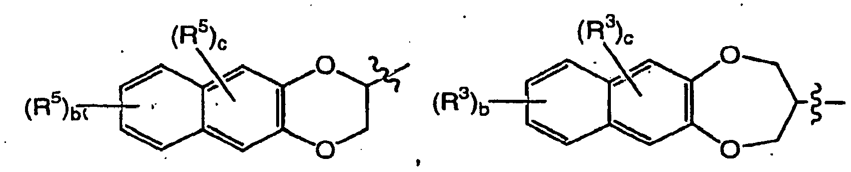 or 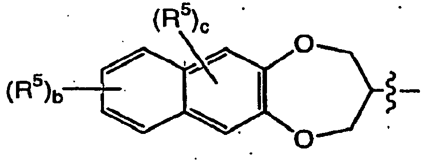 may be similarly prepared according to known methods or for example, according to the processes outlined in Schemes 2 through 5 above, by selecting and substituting the corresponding naphthyl-fused compounds for the benzo-fused starting, materials.

One skilled in the art will further recognize that wherein a single enantiomer (or a mixture of enantiomers wherein one enantiomer is enriched) of a compound of formula (X) is desired, the above processes as described in Schemes 1 through 5 may be applied by substituting the corresponding single enantiomer (or mixture of enantiomers wherein one enantiomer is enriched) for the appropriate starting material.

One skilled in the art will recognize that wherein a reaction step of the present invention may be carried out in a variety of solvents or solvent systems, said reaction step may also be carried out in a mixture of the suitable solvents or solvent systems.

Where the processes for the preparation of the compounds according to the invention give rise to mixture of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their component enantiomers by standard techniques, such as the formation of diastereomeric pairs by salt formation with an optically active acid, such as (-)-di-p-toluoyl-D-tartaric acid and/or (+)-di-p-toluoyl-L-tartaric acid followed by fractional crystallization and regeneration of the free base. The compounds may also be resolved by formation of diastereomeric esters or amides, followed by chromatographic separation and removal of the chiral auxiliary. Alternatively, the compounds may be resolved using a chiral HPLC column.

During any of the processes for preparation of the compounds of the present invention, it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991. The protecting groups may be removed at a convenient subsequent stage using methods known from the art.

The present invention further comprises pharmaceutical compositions containing one or more compounds of formula (I) with a pharmaceutically acceptable carrier. Pharmaceutical compositions containing one or more of the compounds of the invention described herein as the active ingredient can be prepared by intimately mixing the compound or compounds with a pharmaceutical carrier according to conventional pharmaceutical compounding techniques. The carrier may take a wide variety of forms depending upon the desired route of administration (e.g., oral, parenteral). Thus for liquid oral preparations such as suspensions, elixirs and solutions, suitable carriers and additives include water, glycols, oils, alcohols, flavoring agents, preservatives, stabilizers and coloring agents, for solid oral preparations, such as powders, capsules and tablets, suitable carriers and additives include starches, sugars, diluents, granulating agents, lubricants, binders and disintegrating agents. Solid oral preparations may also be coated with substances such as sugars or be enteric-coated so as to modulate major site of absorption. For parenteral administration, the carrier will usually consist of sterile water and other ingredients may be added to increase solubility or preservation. Injectabte suspensions or solutions may also be prepared utilizing aqueous, carries along with appropriate additives.

To prepare the pharmaceutical compositions of this invention, one or more compounds of the present invention as the active ingredient is intimately admixed with a pharmaceutical carrier according to conventional pharmaceutical compounding techniques, which carrier may take a wide variety of forms depending of the form of preparation desired for administration, e.g., oral or parenteral such as intramuscular. In preparing the compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives and coloring agents; for solid oral preparations such as, for example, powders, capsules, caplets, gelcaps and tablets, suitable carriers and additives include starches, sugars, diluents, granulating agents, lubricants, binders and disintegrating agents. Because of their ease in administration, tablets and capsules represent the most advantageous oral dosage unit form, in which case solid pharmaceutical carriers are obviously employed. If desired, tablets may be sugar coated or enteric coated by standard techniques. For parenterals, the carrier will usually comprise sterile water, through other ingredients, for example, for purposes such as aiding solubility or for preservation, may be included. Injectable suspensions may also be prepared, in which case appropriate liquid carriers, suspending agents may be employed. The pharmaceutical compositions herein will contain, per dosage unit, e.g., tablet, capsule, powder, injection, teaspoonful, an amount of the active ingredient necessary to deliver an effective dose as described above. The pharmaceutical compositions herein will contain, per unit dosage unit, e.g., tablet, capsule, powder, injection, suppository, teaspoonful, of from about 0.1-1000 mg and may be given at a dosage of from about 0.01-200.0 mg/kg/day, preferably from abut 0.1 to 100 mg/kg/day, more preferably from about 0.5-50 mg/kg/day, more preferably from about 1.0-25.0 mg/kg/day or any range therein. The dosages, however; may be varied depending upon the requirement of the patients, the severity of the condition being treated and the compound being employed. The use of either daily administration or post-periodic dosing may be employed.

Preferably these compositions are in unit dosage forms from such as tablets, pills, capsules, powders, granules, sterile parenteral solutions or suspensions, metered aerosol or liquid sprays, drops, ampoules, autoinjector devices or suppositories; for oral parenteral, intranasal, sublingual or rectal administration, or for administration by inhalation or insufflation. Alternatively, the composition may be presented in a form suitable for once-weekly or once-monthly administration; for example, an insoluble salt of the active compound, such as the decanoate salt, may be adapted to provide a depot preparation for intramuscular injection. For preparing solid compositions such as tablets, the principal active ingredient is mixed with a pharmaceutical carrier, e.g. conventional tableting ingredients such as com starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective dosage forms such as tablets, pills and capsules. This solid preformulation composition is then subdivided into unit dosage forms of the type described above containing from 0.1 to abut 1000 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.

The liquid forms in which the novel compositions of the present invention may be incorporated for administration orally or by injection include, aqueous solutions, suitably flavored syrups, aqueous or oil suspensions, and flavored emulsions with edible oils such as cottonseed oil, sesame oil, coconut oil or peanut oil, as well as elixirs and similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinyl-pyrrolidone or gelatin.

The treatments described in the present invention may also be carried out using a pharmaceutical composition comprising any of the compounds as defined herein and a pharmaceutically acceptable carrier. The pharmaceutical composition may contain between about 0.1 mg and 1000 mg, preferably about 50 to 500 mg, of the compound, and may be constituted into any form suitable for the mode of administration selected. Carriers include necessary and inert pharmaceutical excipients, including, but not limited to, binders, suspending agents, lubricants, flavorants, sweeteners, preservatives, dyes, and coatings. Compositions suitable for oral administration include solid forms, such as pills, tablets, caplets, capsules (each including immediate release, timed release and sustained release formulations), granules, and powders, and liquid forms, such as solutions, syrups, elixers, emulsions, and suspensions. Forms useful for parenteral administration include sterile solutions, emulsions and suspensions.

Advantageously, compounds of the present invention may be administered in a single daily dose, or the total daily dosage may be administered in divided doses of two, three or four times daily. Furthermore, compounds for the present invention can be administered in intranasal form via topical use of suitable intranasal vehicles, or via transdermal skin patches well known to those of ordinary skill in that art. To be administered in the form of a transdermal delivery system, the dosage administration will, of course, be continuous rather than intermittent throughout the dosage regimen.

For instance, for oral administration in the form of a tablet or capsule, the active drug component can be combined with an oral, non-toxic pharmaceutically acceptable inert carrier such as ethanol, glycerol and water. Moreover, when desired or necessary, suitable binders; lubricants, disintegrating agents and coloring agents can also be incorporated into the mixture. Suitable binders include starch, gelatin, natural sugars such as glucose or beta-lactose, com sweeteners, natural and synthetic gums such as acacia, tragacanth or sodium oleate, sodium stearate, magnesium stearate, sodium benzoate, sodium acetate and sodium chloride. Disintegrators include, without limitation, starch, methyl cellulose, agar, bentonite and xanthan gum.

The liquid forms in suitably flavored suspending or dispersing agents such as the synthetic and natural gums, for example, tragacanth, acacia and methyl-cellulose. For parenteral administration, sterile suspensions and solutions are desired. Isotonic preparations which generally contain suitable preservatives are employed when intravenous administration is desired.

Compounds of this invention may be administered in any of the foregoing compositions and according to dosage regimens established in the art whenever treatment of the present invention are required.

The daily dosage of the products may be varied over a wide range from 0.01 to 200 mg / kg per adult human per day. For oral administration, the compositions are preferably provided in the form of tablets containing, 0.01, 0.05, 0.1, 0.5, 1.0, 2.5, 5.0, 10.0, 15.0, 25.0, 50.0, 100, 150, 200, 250, 500 and 1000 milligrams of the active ingredient for the symptomatic adjustment of the dosage to the patient to be treated. An effective amount of the drug is ordinarily supplied at a dosage level of from about 0.01 mg/kg to about 200 mg/kg of body weight per day. Preferably, the range is from about 0.1 to about 100.0 mg/kg of body weight per day, more preferably, from about 0.5 mg/kg to about 50 mg/kg, more preferably, from about 1.0 to about 25.0 mg/kg of body weight per day. The compounds may be administered on a regimen of 1 to 4 times per day.

Optimal dosages to be administered may be readily determined by those skilled in the art, and will vary with the particular compound used, the mode of administration, the strength of the preparation, the mode of administration, and the advancement of the disease condition. In addition, factors associated with the particular patient being treated, including patient age, weight, diet and time of administration, will result in the need to adjust dosages.

One skilled in the art will recognize that, both *in vivo* and *in vitro* trials using suitable, known and generally accepted cell and / or animal models are predictive of the ability of a test compound to treat or prevent a given disorder.

One skilled in the art will further recognize that human clinical trails including first-in-human, dose ranging and efficacy trials, in healthy patients and / or those suffering from a given disorder, may be completed according to methods well known in the clinical and medical arts.

The following Examples are set forth to aid in the understanding of the invention, and are not intended and should not be construed to limit in any way the invention set forth in the claims which follow thereafter.

### Example 1

### ((3,4-Dihvdro-2H-benzo[b][1,4]dioxepin-3-yl)methyl)sulfamide (Compound #3)

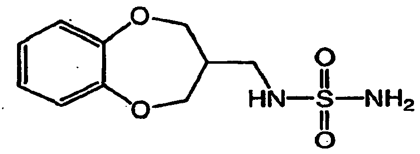

Catechol (5.09 g, 46.2 mmol) and potassium carbonate were combined in acetonitrile and heated to reflux for one hour. 2-Chloromethyl-3-chloro-1-propene (5.78 g, 46.2 mmol) was added and the reaction was continued at reflux for 24 hours. The solution was cooled to room temperature and filtered. The filtrate was evaporated and the residue was diluted with water and extracted with diethyl ether (3 x). The combined organic solution was dried over MgSO₄ and concentrated. Chromatography (2% ethyl ether in hexane) yielded 3-methylene-3,4-dihydro-2H-benzo[b][1,4]dioxepine as a colorless oil.

MS (ESI): 163.2 (M+H⁺)

¹H NMR (300 MHz, CDCl₃), δ: 6.94 (m, 4H), 5.07 (s, 2H), 4.76 (s, 4H).

3-Methylene-3,4-dihydro-2H-benzo[b][1,4]dioxepine (5.00 g, 30.8 mmol) was dissolved in dry THF (100 mL). Borane-THF (1.0 M in THF, 10.3 mL) was added at.0°C. The reaction was stirred at RT for 5 hours. Aminosulfonic acid (6.97 g, 61.6 mmol) was added. The reaction was heated to reflux overnight. The reaction was cooled to room temperature and aqueous sodium hydroxide (3.0 M, 100 mL) was added. The solution was extracted with ethyl acetate (3 x . 1 00 mL). The combined organic solution was dried over MgSO₄. The solution was concentrated under vacuum and purified by chromatography (2% to 8% methanol in dichloromethane) to yield ((3,4-dihydro-2H-benzo[b][1,4]dioxopin-3-yl)methyl)amine as a colorless oil.

MS (ESI): 180.1, (M+H⁺)

¹H NMR (300 MHz, DMSO), δ: 6.92 (m, 4H), 4.21 (m, 2H), 4.07 (m, 2H), 3.33 (broad, 2H), 3.16 (d, *J* = 4 Hz, 1H), 2.72 (d, *J* = 4 Hz, 1H), 2.30 (m, 1H).

((3,4-Dihydro-2H-benzo[b][1,4]dioxepin-3-yl)methyl)amine (2.90-g, 16.2 mmol) and sulfamide (3.11 g, 32.4 mmol) were combined In dry dioxane (60 mi) and heated to reflux overnight. Chloroform was added and the precipitate was removed by filtration. The filtrate was concentrated under vacuum and purified by chromatography (2% to 8% acetone in dichloromethane) to yield the title compound as an off-white solid.

258.8 (M+H⁺)

¹H NMR (300 MHz, DMSO), δ: 6.92 (m, 4H), 6.71 (broad, 1H), 6.59 (broad, 2H), 4.19 (m, 2H), 4.04 (m, 2H), 3.00 (m, 2H), 2.39 (m, 1H).

### Example 2

### N-(2,3-Dihydro-benzo[1,4]dioxin-2-ylmethyl)-sulfamide (Compound #1)

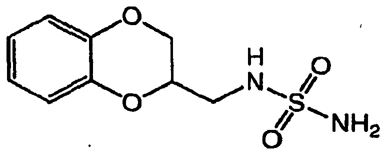

Racemic 2,3-dihydro-1,4-benzdioxin-2-ylmethylamine (4.4 g, 26 mmol) and sulfamide (5.1 g, 53 mmol) were combined in 1,4 dioxane (100 mL) and refluxed for 2 h. The reaction was cooled to room temperature and a small amount of solid was filtered and discarded. The filtrate was evaporated in vacuo and the residue was purified using flash column chromatography (DCM:Methanol - 10:1) to yield a white solid. The solid was recrystallized from DCM to yield the title compound as a white solid.

mp: 97.5 - 98.5°C

| Elemental Analysis: | | | | |
|---|---|---|---|---|
| Anal Calc: | C, 44.25; | H, 4.95; | N, 11.47; | S, 13.13 |
| Anal Found: | C, 44.28; | H, 4.66; | N, 11.21; | S, 13.15 |

H¹ NMR (DMSO d6) □ 6.85 (m, 4H), 6.68 (bd s, 3H, NH), 4.28 (m, 2H), 3.97 (dd, J = 6.9, 11.4 Hz, 1H), 3.20 (m, 1H), 3.10 (m, 1H).

### Example 3

### (Benzo[1,3]dioxol-2-ylmethyl)sulfamide (Compound #2)

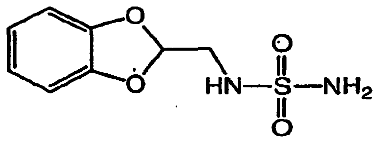

Catechol (10.26 g, 93.2 mmol), sodium methoxide (25% by weight in methanol, 40.3 g, 186 mmol), and methyl dichloroacetate (13.3 g, 93.2 mmol) were combined in dry methanol (100 mL). The solution was heated to reflux overnight. The reaction was cooled to room temperature, acidified by addition of concentrated hydrochloric acid and then reduced in volume under vacuum to about 50 mL. Water was added and the mixture was extracted with diethyl ether (3 x 100 mL). The combined organic solution was dried with MgSO₄, concentrated to a brown solid, and chromatographed (2% ethyl acetate in hexane) to yield benzo[1,3]dioxole-2-carboxylic acid methyl ester as a colorless oil.

MS (ESI): 195.10 (M+H⁺).

¹H NMR (300 MHz, CDCl₃), δ: 6.89 (broad, 4H), 6.29 (s, 1H), 4.34 (q, *J* =7 Hz, 2H), 1.33 (t, *J* =7 Hz, 3H).

To benzo[1,3]dioxole-2-carboxylic acid methyl ester (7.21 g, 40.0 mmol) was added ammonium hydroxide (29% in water, 10 mL) and enough acetonitrile to make the mixture homogeneous (-5 mL). The solution was stirred for two hours at room temperature and then distilled water was added. Benzo[1,3]dioxole-2-carboxylic acid amide precipitated as a white solid and was collected by filtration and used without further purification.

MS (ESI): 160.00 (M+H⁺)

¹H NMR (300 MHz, DMSO), δ: 7.99 (s, broad, 1H), 7.72 (s, broad, 1H), 6.94 (m, 2H) 6.86 (m, 2H), 6.30 (s, 1H).

Benzo[1,3]dioxole-2-carboxylic acid amide (5.44 g, 32.9 mmol) was dissolved in tetrahydrofuran (THF, 100 mL). Lithium aluminum hydride (LAH, 1 M in THF, 39.5 mL, 39.5 mmol) was added slowly to the solution at room temperature. The reaction was stirred at room temperature for 24 hours. Distilled water was added to destroy the excess LAH. Aqueous sodium hydroxide (3.0 M, 100 mL) was added and the solution was extracted with ethyl acetate (3 x 100 mL). The combined organic solution was washed with water and dried over MgSO₄. The solvent was evaporated to yield C-benzo[1,3]dioxol-2-yl-methylamine as a colorless oils

MS (ESI): 152.1 (M+H⁺)

¹H NMR (300 MHz, CDCl₃), δ: 6.87 (m, 4H), 6.09 (t, *J*= 4 Hz, 1H), 3.13 (d, *J*= 4 Hz, 2H)

C-Benzo[1,3]dioxol-2-yl-methylamine (2.94 g, 19.4 mmol) and sulfamide (3.74 g, 38.9 mmol) were combined in dry dioxane (50 mL) and the solution was heated to reflux overnight. The reaction was concentrated and the residue was chromatographed (2% to 10% acetone in dichloromethane) to yield the title compound as a white solid.

MS (ESI): 230.0 (M+H⁺)

¹H NMR (300 MHz, CDCl₃), δ: 6.87 (m, 4H), 6.25 (t, *J* = 4 Hz, 1H), 4.79 (broad, 1H), 4.62 (broad, 1H), 3.64 (d, *J* = 4 Hz, 2H).

### Example 4 _{.}

### (2S)-(-)-N-(2,3-Dihydro-benzo[1,4]dioxin-2-ylmethyl)-sulfamide (Compound #41

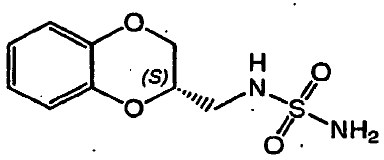

Catechol (13.2 g, 0.12 mol) and potassium carbonate (16.6 g, 0.12 mol) were stirred in DMF (250 mL) and (2R)-glycidyl tosylate (22.8 g, 0.10 mod) was added and the reaction was stirred at 60°C for 24 h. The reaction was cooled to room temperature and diluted with ice water (1 L) and extracted with diethyl ether (4 times). The combined organic solution was washed 3 times with 10% - potassium carbonate, once with water, once with brine and evaporated in vacuo to yield a white solid which was purified by flash column chromatography (DCM:Methanol - 50:1) to yield ((2S)-2,3-dihydro-benzo[1,4]dioxin-2-yl)-methanol as a solid.

The solid (13.3 g, 68 mmol) was dissolved in pyridine (85 mL) cooled to 0°C, p-toluenesulfonyl chloride (13.0 g, 68 mmol) was added and the reaction mixture stirred at room temperature for 20h. The reaction was diluted with diethyl ether (1 L) and 1 N HCl (1.2 L). The organic layer was separated and washed 2 times with 1N HCl (500 mL), 4 times with water (150 mL), once with brine, dried (MgSO₄) and evaporated in vacuo to yield a white solid which was purified by flash column chromatography (Hept:Fr4 - 2:1) to yield toluene-4-sulfonic-acid (2S)-2,3-dihydro-benzo[1,4]dioxin-2-ylmethyl ester as a white solid.

The white solid was combined with potassium phthalimide (14.4 g, 78 mmol) in DMF (250 mL) and heated to reflux for 1 h, cooled to room temperature and poured into vigorously stirring water (1.5 L) and stirred 30 min. White solid was filtered and the solid was washed several times with water, 2% NaOH, and water again and let air dry to yield a (2S)-2-(2,3-Dihydro-benzo[1,4]dioxin-2-ylmethyl)-isoindole-1,3-dione as white powdery solid.

The powdery white solid was combined with hydrazine (2.75 g; 86 mmol) in EtOH (225 mL) and heated at reflux for 2 h, cooled to room temperature and 1N HCl added to pH 1.0 and stirred for 15 min. White solid was filtered and washed with fresh EtOH (solid discarded) and the filtrate was evaporated in vacuo to a solid, which was partitioned between diethyl ether and dilute aqueous NaOH. The diethyl ether solution was dried (Na₂SO₄) and evaporated in vacuo to a yield a light yellow oil. The oil was purified by flash column chromatography (DCM:MeOH -10:1) to yield an oil. A portion of the oil (4.82 g, 29 mmol) in 2-propanol (250 mL) was treated with 1 N HCl (30 mL) and heated on steambath until homogeneous and then let cool to room temperature. After 3 tri, the mixture was ice cooled for 2 h. A white flaky solid (the corresponding HCl salt of (2S)-C-(2,3-Dihydro-benzo[1,4]dioxin-2-yl)-methylamine) was filtered off and then recrystallized again from 2-propanol to yield a white solid.

[α]_{D} = -69.6 (c = 1.06, EtOH)

The white solid was partitioned between DCM and dilute NaOH, and the DCM was dried (NaSO₄) and evaporated in vacuo to yield (2S)-C-(2,3-Dihydro-benzo[1,4]dioxin-2-yl)-methylamine as an oil.

[α]_{D} = -57.8 (c = 1.40, CHCl₃)

The oil (2.1 g, 12.7 mmol) and sulfamide (2.44 g, 25.4 mmol) were refluxed in dioxane (75 mL) for 2 h and the crude product was purified by flash column chromatography (DCM:MeOH 10: 1) to yield a white solid, which was recrystallized from DCM to yield the title compound as a white crystalline solid.

mp 102-103°C

[α]_{D} = -45.1° (c = 1.05, M);

¹H NMR (DMSOd6) δ 6.86 (m, 4H), 6.81 (bd s, 3H, NH), 4.3 (m, 2H), 3.97 (dd, J = 6.9, 11.4 Hz, 1H), 3.20 (dd, J = 5.5, 13.7 Hz, 1H), 3.10 (dd, J = 6.9, 13.7 Hz, 1H)

| Elemental Analysis: | | | | |
|---|---|---|---|---|
| Anal Calc: | C, 44.25; | H, 4.95; | N, 11.47; | S, 13.13 |
| Anal Found: | C, 44.20; | H, 4.69; | N, 11.40; | S, 13.22. |

### Example 5

### N-(2,3-Dihydro-benzo[1,4]dioxin-2-ylmethyl)-N',N' dimethylsulfamide (Compound #6)

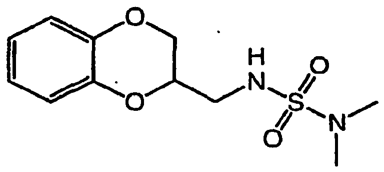

Racemic 2,3-dihydro-1,4-benzdioxin-2-ylmethylamine (8.25 g, 5.0 mmol) and triethylamine (1.52 g, 15 mmol) were combined in DMF (10 mL) and cooled in an ice bath as dimethylsulfamoyl chloride (1.44 g, 10 mmol) was added. The reaction mixture was then stirred for 3 hr with continued cooling. The reaction mixture was partitioned between ethyl acetate and water, and the ethyl acetate solution was washed with brine, dried (MgSO₄) and evaporated in vacuo to yield an oil. The oil was purified using flash column chromatography (ethyl acetate:Heptane - 1:1) to yield a white solid, which was recrystallized (ethyl acetate/Hexane) to yield the title compound as a white floccular solid.

mp 76 - 78°C

MS 273 (MH⁺)

| Elemental Analysis: | | | | |
|---|---|---|---|---|
| Anal Calc: | C, 48.52; | H, 5.92; | N, 10.29; | S, 11.78 |
| Anal Found: | C, 48.63; | H, 5.62; | N, 10.20; | S, 11.90 |

¹H NMR (CDCl₃) δ 6.87 (m, 4H), 4.59 (bd m, 1H, NH), 4.35.(m, 1H), 4.27 (dd, J = 2.3, 11.4 Hz, 1H), 4.04 (dd, J = 7.0, 11.4, 1H), 3.36 (m, 2H), 2.82 (s, 6H).

### Example 6

### N-(2,3-Dihydro-benzo[1,4]dioxin-2-ylmethyl)-N-methylsulfamide (Compound #7)

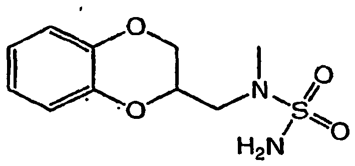

Racemic 2,3-dihydro-1,4-benzdioxin-2-ylmethylamine (825 mg, 5 mmol) was dissolved in ethyl formate (15 mL), refluxed for 30 min and evaporated in vacuo to yield N-(2,3-dihydro-benzo[1,4]dioxin-2-ylmethyl)-formamide as an oil.

The oil in diethyl ether (25 mL) was treated with 1 M LAH in THF (9.0 mL, 9.0 mmol) at 0°C and stirred for 5 h at room temperature. The reaction was cooled in an ice bath and quenched with water (0.50 mL), followed by 3 N NaOH (0.50 mL) and water (0.50 mL). The mixture was then stirred at room temperature for 1 h. Solid was filtered and the filtrate was evaporated in vacuo to yield a residue which was partitioned between 1N HCl and diethyl ether. The aqueous phase was basified with 1N NaOH and extracted with diethyl ether. The organic phase was dried (MgSO₄) and evaporated in vacuo to yield (2,3-dihydro-benzo[1,4]dioxin-2-ylmethyl)-methyl-amine as an oil.

MS 180 (MH⁺)

¹H NMR (CDCl₃) δ 6.85 (m, 4H), 4.30 (m, 2H), 4.02 (dd, J = 7.9, 11.6 Hz, 1H), 2.85 (m, 2H), 2.50 (s, 3H)

The oil (380 mg, 2.1 mmol) and sulfamide (820 mg, 8.5 mmol) were combined in dioxane (15 mL), refluxed for 1.5 h and evaporated in vacuo to yield a crude residue. The residue was purified via column chromatography (ethyl acetate/Heptane 1:1) and the resultant solid was recrystallized from ethyl acetate/Hexane to yield the title compound as a white solid.

mp 97-98°C

MS 257 (M⁻¹)

| Elemental Analysis: | | | | |
|---|---|---|---|---|
| Anal Calc: | C, 46.50; | H, 5.46; | N, 10.85; | S, 12.41 |
| Anal Found: | C, 46.48; | H, 5.65; | N, 10.90; | S, 12.07 |

¹H NMR (CDCl₃) δ 6.86 (m, 4H), 4.52 (bs, 2H), 4.46 (m, 1H), 4.29 (dd, J = 2.3, 11.5 Hz, 1H), 4.05 (dd, J = 6.5, 11.5 Hz, 1H), 3.51 (dd, J = 6.7, 14.9 Hz, 1H), 3.40 (dd, J = 5.9, 14.9 Hz, 1H), 2.99 (s, 3H).

### Example 7

### (2S)-(-)(N-(6-Chloro-2,3-dihydro-benzo[1,4]dioxin-2-ylmethyl)-sulfamide (compound #8)

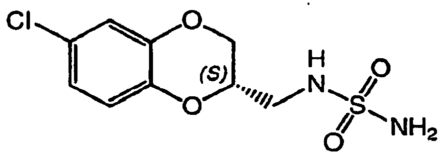

Following the procedure outlined in Example 4 above, 4-chlorocatechol was reacted to yield a mixture of (2S)-C-(7-Chloro-2,3-dihydro-benzo[1,4]dioxin-2-yl)-methylamine and (2S)-C-(6-Chloro-2,3-dihydro-benzo[1,4]dioxin-2-yl)-methylamine (ca. 3:1 ratio of 6-chloro:7-chloro isomers by RP HPLC).

The mixture was dissolved in 2-propanol (100 mL) and 1N HCl in diethyl ether was added until pH = 1.0 was attained. The hydrochloride salt that precipitated was filtered (2.65 g) and re-crystallized from methanol/IPA to yield white crystals. The white crystals were partitioned between DCM and dilute NaOH. The DCM was dried and evaporated in vacuo to yield purified (2S)-C-(6-Chloro-2,3-dihydro-benzo[1,4]dioxin-2-yl)-methylamine as an oil.

[α]_{D} = -67.8 (c = 1.51, CHCl₃)

The oil (7.75 mmol) and sulfamide (1.50 g, 15.5 mmol) were combined in dioxane (50 mL) and refluxed for 2.0 h, cooled to room temperature and evaporated in vacuo to yield a solid. The product was purified via flash column using DCM/methanol 20:1 to yield the title compound as a white solid.

MS 277 (M⁻¹)

[α]_{D} = -59.9° (C = 1.11, M)

¹H NMR (CDCl₃) δ 6.90 (d, J = 2.2 Hz, 1H), 6.81 (m, 2H), 4.76 (m, 1H), 4.55 (s, 2H), 4.40 (m, 1H), 4.29 (dd, J = 2.4, 11.5 Hz, 1H), 4.05 (dd, J = 7.1, 11.5 Hz, 1H), 3.45 (m, 2H)

| Elemental Analysis: | | | |
|---|---|---|---|
| Anal Calc: | C, 38.78; | H, 3.98; | N, 10.05 |
| Anal Found: | C, 38.80; | H, 3.67; | N, 9.99. |

The filtrates of the crystallized hydrochloride salt of (2S)-C-(6-Chloro-2,3-dihydro-benzo[1,4]dioxin-2-yl)-methylamine prepared above were recovered (ca. 1:1 of 6-chloro:7-chloro isomers) and evaporated in vacuo to yield a solid, which was partitioned between DCM (200 mL) and dilute NaOH (0.5 M. 50 mL). The DCM solution was washed once with brine, dried (Na₂SO₄) and evaporated in vacuo to yield an oil, which was purified via reverse phase HPLC (10 - 50% ACN with 0.16% TFA in water with 0.20% TFA) to yield (2S)-C-(7-Chloro-2,3-dihydro-benzo[1,4]dioxin-2-yl)-methylamine as a residue.

The residue was combined with sulfamide (0.90 g, 9.4 mmol) in dioxane (25 mL) and refluxed for 2.5 h, cooled to room temperature and evaporated in vacuo to yield an oil. The oil was purified by flash column chromatography using DCM/methanol - 10:1 to yield (2S)-(-)-N-(7-Chloro-2,3-dihydro-benzo[1,4]dioxin-2-ylmethyl)-sulfamide as a white solid.

MS 277 (M⁻¹)

¹H NMR (CDCl₃/CD₃OD) δ 6.88 (d, J = 0.7 Hz, 1H), 6.81 (m, 2H), 4.37 (m, 1H), 4.30 (dd, J = 2.3, 11.6 Hz, 1H), 4.04 (dd, J =7.0, 11.6 Hz, 1H), 3.38 (m, 2H).

### Example 8

### Chroman-2-ylmethylsulfamide (Compound #10)

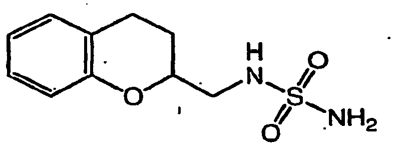

Chroman-2-carboxylic acid (4.5 g, 25 mmol) and HOBT (3.86 g, 25 mmol) were combined in DCM (40 mL) and DMF (10 mL). Dimethylaminopropyl ethylcarbodiimide (EDC, 4.84 g, 25 mmol) was added at room temperature and the reaction mixture was stirred for 30 min. Ammonium hydroxide (2.26 mL, 33.4 mmol) was added and the reaction mixture was stirred for 16h. The reaction mixture was diluted with DCM (50 mL) and water (50 mL) and the pH of the mixture was adjusted to about pH = 3.0 with 1N HCl. The DCM was separated and the aqueous phase extracted twice with DCM. The combined DCM phase was dried (Na₂SO₄) and evaporated in vacuo to yield an oil, which was purified with flash column chromatography (ethyl acetate) to yield an oil.

The oil (5.35 g, 30 mmol) in THF (90 mL) was stirred as 1 M LAH in THF (36 mL, 36 mmol) was added and the reaction mixture was then stirred at room temperature for 20 h. The reaction was quenched with water, stirred for 2 hours, the solution decanted, dried (Na₂SO₄) and evaporated in vacuo to yield C-chroman-2-yl-methylamine as an oily amine.

The oily amine (1.63 g, 10 mmol) and sulfamide (1.92 g, 20 mmol) were combined in dioxane (50 mL) and brought to reflux for 2 h. The solution was cooled and evaporated in vacuo to yield an oil, which was purified via column chromatography (DCM:Methanol 10:1) to yield a white solid. The solid was recrystallized from ethyl acetate/hexane to yield chroman-2-ylmethylsulfamide as a white solid.

mp 100-101°C

MS 241 (M⁻¹)

| Elemental Analysis: | | | | |
|---|---|---|---|---|
| Anal Calc: | C, 49.57; | H, 5.82; | N, 11.56; | S, 13.23 |
| Anal Found: | C, 49.57; | H, 5.80; | N, 11.75; | S, 13.33. |

### Example 9

### 2-(2,3-Dihydro-benzo[1,4]dioxin-2-yl)-ethylsulfamide (Compound #16)

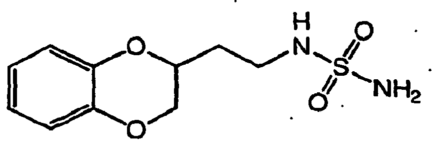

Potassium cyanide (2.05 g, 31.5 mmol) was added to 2-bromomethyl-(2,3 dihydrobenzo[1,4]dioxine) (6.87 g, 30 mmol) in DMSO (90 mL) and stirred at ambient temperature for 20 h. The reaction mixture was then diluted with water (250 mL) and extracted twice with diethyl ether. The diethyl ether was washed with water, then washed twice with brine, dried (Na₂SO₄) and evaporated in vacuo to yield 2-cyanomethyl-(2,3 dihydrobenzo[1,4]dioxine) as a white solid.

¹H NMR (CDCl₃) δ 6.89 (m, 4H), 4.50 (m, 1H), 4.31 (dd, J = 2.3, 11.5 Hz, 1H), 4.08 (dd, J = 6.2, 11.6 Hz, 1H), 2.78 (d, J = 6.1, Hz, 2H)

The 2-cyanomethyl-(2,3 dihydrobenzo[1,4]dioxine) was dissolved in THF (50 mL) and 1 M BH₃ in THF (80 mL, 80 mmol) was added and the reaction mixture refluxed for 5 h, then stirred at ambient temperature for 16h. With ice bath cooling, 2N HCl was added until pH = 1.0 was achieved. The reaction mixture was then stirred for 1 h at room temperature and evaporated in vacuo to yield an oil. The oil was partitioned between 3N NaOH and diethyl ether, and the diethyl ether solution was washed with brine, dried (Na₂SO₄) and evaporated in vacuo to yield crude 2-(2,3 dihydrobenzo[1,4]dioxin-2-yl)ethylamine.

MS (M+H)⁺ 180.

The crude 2-(2,3 dihydrobenzo[1,4]dioxin-2-yl)ethylamine in dioxane (100 mL) was combined with sulfamide (3.0 g, 31 mmol) and heated to reflux for 2 h. The solution was cooled and evaporated in vacuo to yield an orange solid, which was purified by column chromatography (DCM:MeOH - 10:1) to yield a white solid. The solid was re-crystallized from DCM to yield the title compound as a solid.

MS (M-1) 257

MP 101 - 103°C (corr)

¹H NMR (CDCl₃): δ 6.86 (m, 4H), 4.70 (m, 1H), 4.52 (s, 2H), 4.30 (m, 2H), 3.94 (dd, J = 7.4, 11.3 Hz, 1H), 3.43 (dd, J = 6.4, 12.9 Hz, 2H), 1.94 (dd, J = 6.5, 12.9, 2H).

| Elemental Analysis: | | | | |
|---|---|---|---|---|
| Measured: | C, 46.48; | H, 5.60; | N, 10.81; | S, 12.41 |
| Calculated: | C, 46.50; | H, 5.46; | N, 10.85; | S, 12.41 |

### Example 10

### (2S)-(-)-N-(6,7 Dichloro-2,3-dihydro-benzo[1,4]dioxin-2-ylmethyl)-sulfamide (Compound #29)

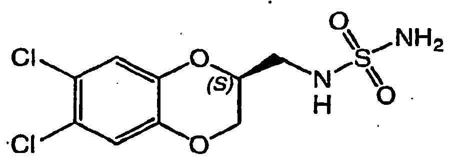

4,5 Dichloroatechol (8.6 g, 48 mmol) and potassium carbonate (6.64 g, 48 mmol) were stirred in DMF (200 mL). (2R)-Glycidyl tosylate (9.12 g, 40 mmol) was added and the reaction mixture was stirred at 60°C for 24 h. The reaction mixture was cooled to room temperature and then diluted with ice water (600 mL) and extracted with diethyl ether (4 times). The combined organic solution was washed 3 times with 10% potassium carbonate, twice with brine, dried (MgSO₄) and evaporated in vacuo to yield a viscous oil of (2S)-2-(6,7-dichloro-2,3-dihydro-benzo[1,4]dioxine) methanol.

The (2S)-2-(6,7 dichloro-2,3-dihydro-benzo[1,4]dioxine) methanol oil (6.4 g, 27 mmol) was dissolved in pyridine (50 mL) cooled to 0°C. Then, p-toluenesulfonyl chloride (5.2 g, 27 mmol) was added and the reaction mixture was stirred at room temperature for 20h. The reaction mixture was diluted with diethyl ether and 1N HCl (750 mL) and the organic layer was separated and washed 2 times with 1N HCl (250 mL), once with water (150 mL), twice with brine, dried (MgSO₄) and evaporated in vacuo to yield light yellow solid of toluene-4-sulfonic acid (2S)-6,7-dichloro-2,3-dihydro-benzo[1,4]dioxin-2-ylmethyl ester.

¹H NMR (CDCl3): δ 7.79 (d, J = 8.3 Hz, 2H), 7.36 (d, J = 8.0 Hz, 2H), 6.94 (s, 1H), 6.83 (s, 1H), 4.37 (m, 1H), 4.2 (m, 3H), 4.03 (dd, J = 6.3, 11.7 Hz, 1H), 2.47 (s, 3H).

Toluene-4-sulfonic acid (2S)-6,7-dichloro-2,3-dihydro-benzo[1,4]dioxin-2-ylmethyl ester (8.0 g, 20.5 mmol) was combined with potassium phthalimide (6.1 g, 33 mmol) in DMF (75 mL) and heated to reflux for 1 h, cooled to room temperature and poured into vigorously stirring water (0.5 L) and then stirred 30 min. White solid was filtered and the solid was washed several times with water, 2% NaOH, and water again and then let air dry to yield (2S)-2-(6,7-dichloro-2,3-dihydro-benzo[1,4]dioxin-2-ylmethyl)-isoindole-1,3-dione (6.0 g, 80%) as a white powdery solid.

The white powdery solid was combined with hydrazine (1.06 g, 33 mmol) in EtOH (80 mL) and heated at reflux for 2 h, then cooled to room temperature. 1N HCl was added to adjust the reaction mixture's pH to pH 1.0 and the reaction mixture was then stirred for 15 min. White solid was filtered and washed with fresh EtOH (solid discarded) and the filtrate was evaporated in vacuo to a solid, which was partitioned between diethyl ether and dilute aqueous NaOH. The diethyl ether solution was dried (Na₂SO₄) and evaporated in vacuo to a yield a viscous oil of (2S)-2-aminomethyl-(6,7-dichloro-2,3-dihydro-benzo[1,4]dioxine).

¹H NMR (CDCl3): δ 6.98 (s, 1H), 6.96 (s, 1H), 4.25 (dd, J = 2.0, 11.2 Hz, 1H), 4.15 (m, 1H), 4.0 (m, 1H), 2.97 (d, J = 5.5 Hz, 2H)

A portion of the oil (3.8 g, 16 mmol) and sulfamide (3.1 g, 32.4 mmol) were refluxed in dioxane (100 mL) for 2 h and the crude product was purified by flash column chromatography (DCM:MeOH 20:1) to yield the title compound as a white solid, which was recrystallized from ethyl acetate / hexane to yield the title compound as a white crystalline solid.

MS [M-H]⁻ 311.0

mp 119-121°C

[α]_{D} = -53.4°(c=1.17,M)

¹H NMR (DMSOd6): δ 7.22 (s, 1H), 7.20 (s, 1H), 6.91 (bd s, 1H), 6.68 (bd s, 2H), 4.35 (m, 2H), 4.05 (dd, J = 6.5, 11.5 Hz, 1H), 3.15 (m, 2H) Elemental Analysis:

| Elemental. Analysis: | | | | | |
|---|---|---|---|---|---|
| Measured: | C, 34.52; | H, 3.22; | N, 8.95; | Cl, 22.64; | S, 10.24 |
| Calculated: | C, 34.64; | H, 2.68; | N, 8.87; | Cl, 22.94; | S, 10.35. |

### Example 11

### (2S)-(-)-N-(7-Amino-2,3-dihydro-benzo[1,4]dioxin-2-ylmethyl)-sulfamide (Compound #36)

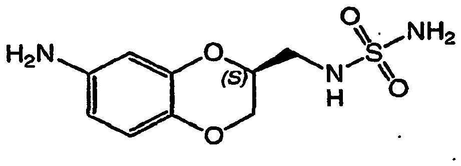

(2S)-(-)-N-(2,3-Dihydro-7-nitro-benzo[1,4]dioxin-2-ylmethyl)-sulfamide (1.2 g, 4.15 mmol), was prepared from 4-nitrocatechol according to the process outlined in Example 4. The (2S)-(-)-N-(2,3-Dihydro-7-nitro-benzo[1,4]dioxin-2-ylmethyl)-sulfamide, was then combined with 10% Pd/C in methanol (120 mL) and shaken under hydrogen atmosphere (39 psi) at room temperature for 3 h. The solids were filtered and washed with 10% M in DCM and the filtrate was evaporated in vacuo to yield crude product. The crude product was dissolved in 0.2 N HCl (25 mL), frozen and lyophilized to yield the title compound as a white flaky solid, as the corresponding hydrochloride salt.

MS (M+H)⁺ 260

¹H NMR (DMSO d6): δ 10.2 (bd s, 3H), 6.86 (m, 1H), 6.85 (s, 1H), 6.74 (dd, J = 2.5, 8.4 Hz, 1H), 4.22 (m, 2H), 3.88 (dd, J = 6.7, 11.4 Hz, 1H), 3.04 (m, 2H)

### Example 12

### (2S)-(-)-N-(7-Methyl-2,3-dihydro-benzo[1,4]dioxin-2-ylmethyl)-sulfamide (Compound #19)

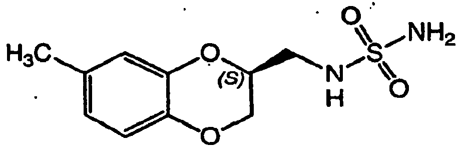

Title compound was prepared according to the procedure described in Example 4 above, starting with 4-methylcatechol, to yield a white solid, which was recrystallized from ethyl acetate/ hexane to yield the title compound as a white solid.

MS [M-H]⁻ 257

¹H NMR (CDCl3): δ 6.76 (m, 1H), 6.66 (m, 2H), 4.80 (m, 1H), 4.57 (bd s, 1H), 4.40 (m, 1H), 4.28 (m, 1H), 4.03 (dd, J = 6.9, 11.4 Hz, 1H), 3.45 (m, 2H), 2.25 (s, 3H).

| Elemental Analysis | | | | |
|---|---|---|---|---|
| Calculated: | C, 46.50; | H, 5.46; | N, 10.85; | S, 12.41 |
| Found: | C, 46.65; | H, 5.60; | N, 10.84; | S, 12.61. |

### Example 13

### Cell Titer Glow / Cell Viability In Vitro Assay

The assay was run according to the procedure listed within the insert of the kit purchased from Promega.

Cell Cultures were prepared as follows. Dissociated hippocampal and cortical cell cultures were established from embryonic day 18 rat fetuses. The fetuses were removed via cesarean section from pregnant dams (Harlan Sprague-Dawley) anesthetized with halothane according to the AVMA Panel on Euthanasia. Pups were decapitated and the brains were removed and placed in Hank's Balanced Salt solution (1x HBSS; Gibco, Rockville,MD). The hippocampi and cortices were dissected out and pooled according to tissue-type. Tissue was trypsinized for 15 min (1mg/ml trypsin-HBSS; Worthington, Lakewood, NJ), rinsed with fresh HBSS, incubated in trypsin inhibitor (1mg/ml; Sigma, St. Louis, MO) for 5 min, rinsed again with fresh HBSS and then triturated in 1ml fresh HBSS with a fire-polished glass pipette. Dissociated cells were seeded at 10,000 cells/well onto poly-D-lysine coated 96-well plates (BD BioScience, Bedford, MA) containing 100ul/well Eagle's Minimal Essential Media (MEM; Gibco) supplemented with 26mM NaHCO₃ (Sigma), 40mM glucose (Sigma), 20mM KCI (Sigma), 1 mM sodium pyruvate (Sigma), 10% (v/v) heat-inactivated fetal bovine serum (Hyclone, Logan, UT), and 0.001% gentamicin sulfate (Sigma) (pH 7.4). Serum-free cultures were plated and maintained in Neurobasal medium + B27 supplement (Gibco). Cells were allowed to attach for 24h in a humidified 37°C 5% CO₂ incubator before experimental treatment.

Test compounds were prepared as follows: A 10mM stock in DMSO of each compound was diluted 1:50 in DPBS rendering a final stock of 200 µM. The stock was further diluted in DPBS to obtain a final concentration of 0.1, 1 and 120 µM compound within each 100uL well. Equal amounts of vehicle or diluted compound were added to each culture well.

A 30% stock solution of hydrogen peroxide (H₂O₂; Sigma) was diluted with DPBS 1:100 to yield a 3mM stock. Five microliters of vehicle or H₂O₂ stock solution was added to each 100ul culture well to yield a final concentration of 150uM.

Compound #8 was tested according to the procedure as described herein, with results as listed in Table 4 and 5 below. Note that in the data listed below, each plate was run in triplicate for a total of n = 9.

**Table 4: Rat Hippocampal Cultures, insult with 150µM H₂O₂**

| | **Vehicle** | **Compound #8** | **Compound #8** | **Compound #8** |
|---|---|---|---|---|
| | | **0.1 µM** | **1 µM** | **10 µM** |
| Plate 1 | 0.5 | 24 | 16 | 10 |
| Plate 2 | 0.6 | 26 | 17 | 14 |
| Plate 3 | 0.5 | 24 | 17 | 15 |
| Mean | 0.5 | 25** | 17 | 13 |
| Standard Deviation | 0.1 | 1 | 0.6 | 3 |

**Table 5: Rat Cerebral Cortical Cultures, insult with 150µM H₂O₂**

| | **Vehicle** | **Compound #8** | **Compound #8** | **Compound #8** |
|---|---|---|---|---|
| | | **0.1 µM** | **1 µM** | **10 µM** |
| Plate 1 | 1.2 | 28 | 19 | 10 |
| Plate 2 | 1 | 64 | 51 | 18 |
| Plate 3 | 0.8 | 97 | 38 | 51 |
| Mean | 1 | 63* | 36 | 26 |
| Standard Deviation | 0.2 | 35 | 16 | 22 |

| | | | | |
|---|---|---|---|---|
| Kruskal-Wallis multiple comparisons test with Dunn's post-hoc test; *, p<0.05 **, p<0.01 | | | | |

Thus, the data show that Compound #8 was effective at protecting neurons from death and / or damage from oxidative stress or oxidative injury, for example as a result of oxygen / peroxide radicals.

### Example 14

As a specific embodiment of an oral composition, 100 mg of the Compound #8 prepared as in Example 7 is formulated with sufficient finely divided lactose to provide a total amount of 580 to 590 mg to fill a size O hard gel capsule.

## Claims

1. A compound of formula (I) 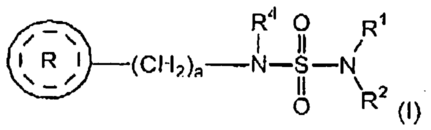 or a pharmaceutically acceptable salt thereof for use in neuroprotection, wherein
R¹ and R² are each independently selected from the group consisting of hydrogen and lower alkyl;
R¹ is selected from the group consisting of hydrogen and lower alkyl:
a is an integer from 1 to 2:
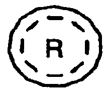 is selected from the group consisting of 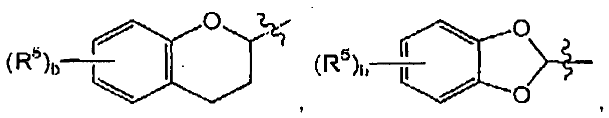 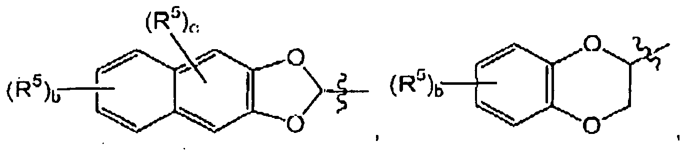 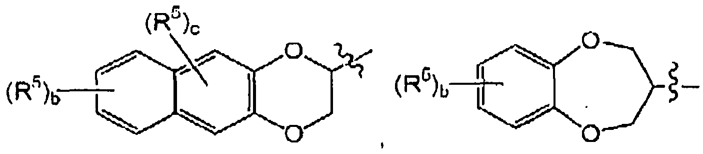 and 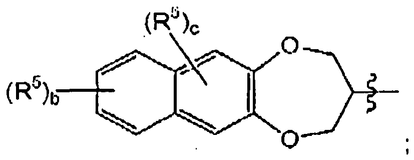 wherein b is an integer from 0 to 4; and wherein c is an integer from 0 to 2;
each R⁵ is independently selected from the group consisting of halogen, lower alkyl and nitro;
wherein "lower alkyl" refers to a carbon chain composition of 1-4 carbon atoms,
provided that when 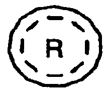 is 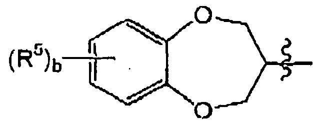 or 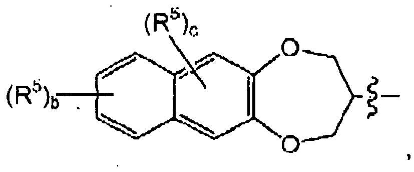 then a is 1.

2. A compound of formula (I) 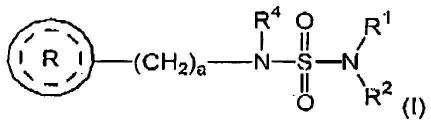 or a pharmaceutically acceptable salt thereof for use in the treatment of an acute neurodegenerative disorder,
wherein
R¹ and R² are each independently selected from the group consisting of hydrogen and lower alkyl;
R⁴ is selected from the group consisting of hydrogen and lower alkyl; a is an integer from 1 to 2;
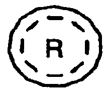 is selected from the group consisting of 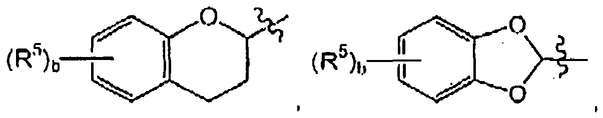 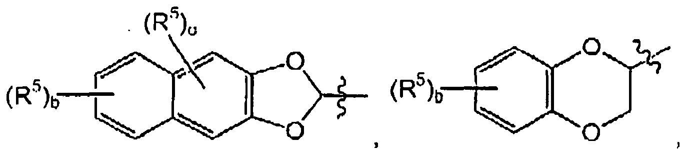 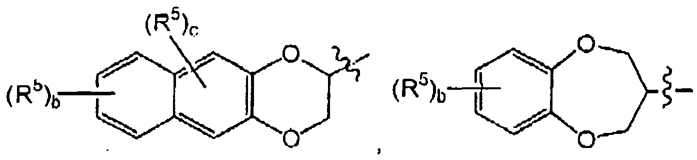 and 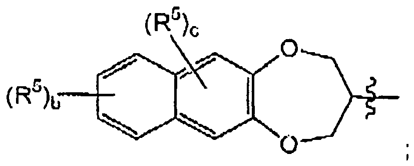 wherein b is an integer from 0 to 4; and wherein c is an integer from 0 to 2;
each R⁵ is independently selected from the group consisting of halogen, lower alkyl and nitro;
wherein "lower alkyl" refers to a carbon chain composition of 1-4 carbon atoms,
provided that when 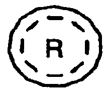 is 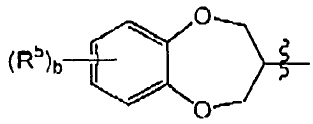 or 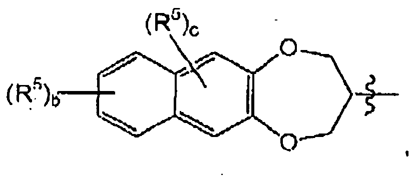 then a is 1.

3. A compound of formula (I) 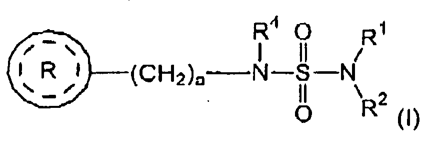 or a pharmaceutically acceptable salt thereof for use In the treatment of a chronic neurodegenerative disorder,
wherein
R¹ and R² are each independently selected from the group consisting of hydrogen and lower alkyl;
R⁴ is selected from the group consisting of hydrogen and lower alkyl;
a is an integer from 1 to 2;
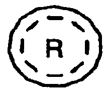 is selected from the group consisting of 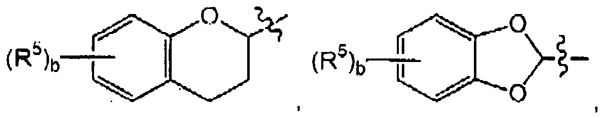 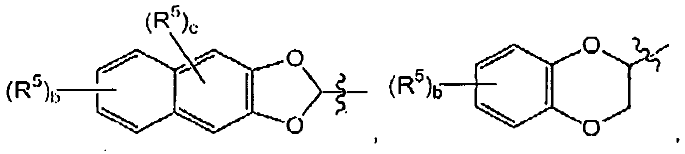 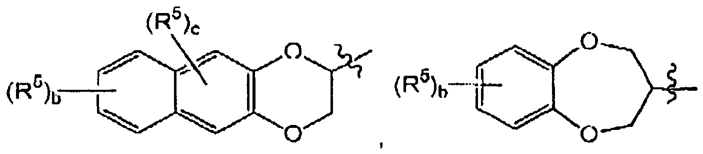 and 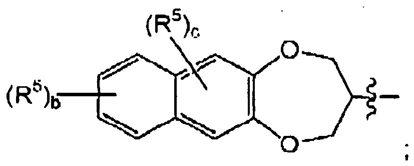 wherein b is an integer from 0 to 4; and wherein c is an integer from 0 to 2;
each R⁵ is independently selected from the group consisting of halogen, lower alkyl and nitro;
wherein "lower alkyl" refers to a carbon chain composition of 1-4 carbon atoms,
provided that when 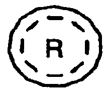 is 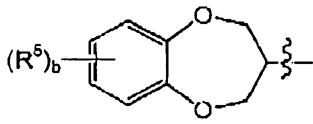 or 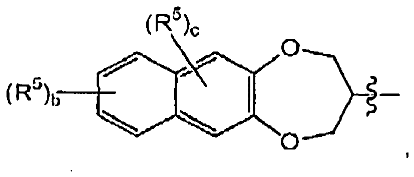 then a is 1.

4. A compound of formula (I) 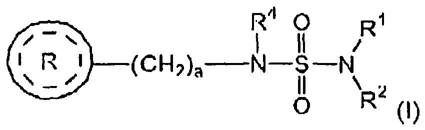 or a pharmaceutically acceptable salt thereof for use in the prevention of neuron death or damage following brain, head or spinal cord trauma or injury,
wherein
R¹ and R² are each independently selected from the group consisting of hydrogen and lower alkyl;
R⁴ is selected from the group consisting of hydrogen and lower alkyl;
a is an integer from 1 to 2;
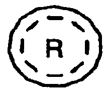 is selected from the group consisting of 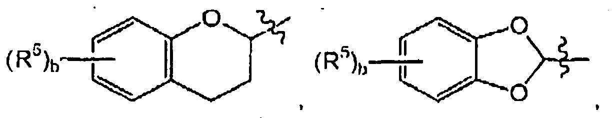 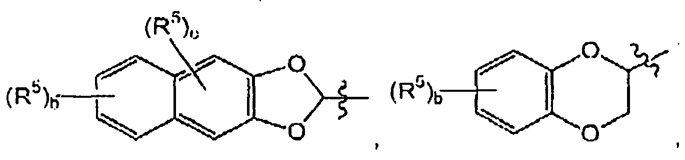 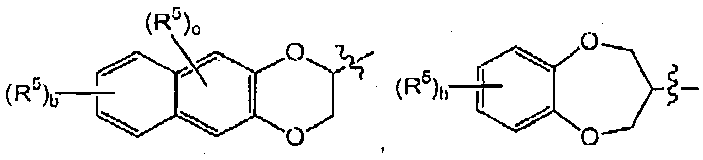 and 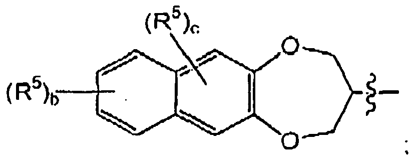 wherein b is an integer from 0 to 4; and wherein c is an integer from 0 to 2;
each R⁵ is independently selected from the group consisting of halogen, lower alkyl and nitro;
wherein "lower alkyl" refers to a carbon chain composition of 1-4 carbon atoms,
provided that when 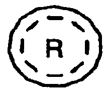 is 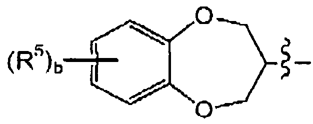 or 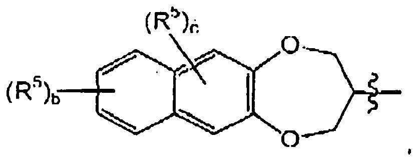 then a is 1.

5. The compound or a pharmaceutically acceptable salt thereof as in any of Claims 1 to 4, wherein
R¹ and R² are each independently selected from the group consisting of hydrogen and lower alkyl;
R⁴ is selected from the group consisting of hydrogen and lower alkyl;
a is an integer from 1 to 2;
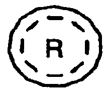 is selected from the group consisting of 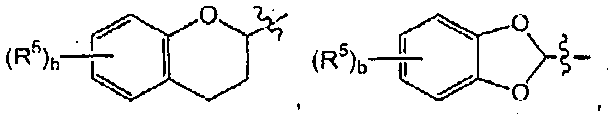 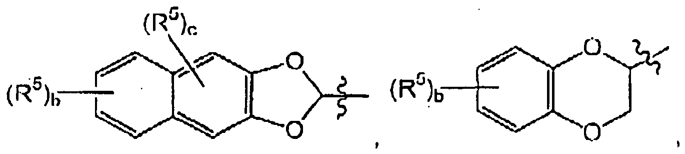 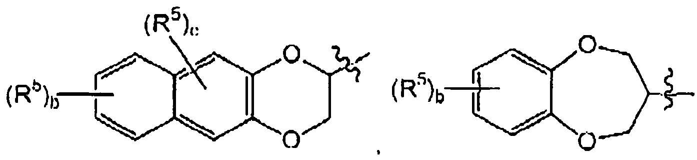 and 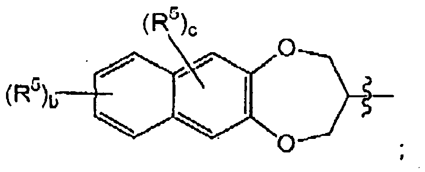 wherein b is an integer from 0 to 2; and wherein c is an integer from 0 to 1;
each R⁵ is independently selected from the group consisting of halogen, lower alkyl and nitro;
wherein "lower alkyl" refers to a carbon chain composition of 1-4 carbon atoms,
provided that when 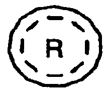 is 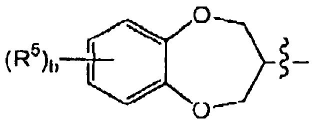 or 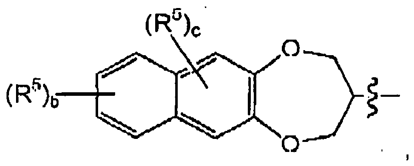 then a is 1.

6. The compound or a pharmaceutically acceptable salt thereof as in Claim 5, wherein
R¹ and R² are each independently selected from the group consisting of hydrogen and lower alkyl;
R⁴ is selected from the group consisting of hydrogen and lower alkyl;
a is an integer from 1 to 2;
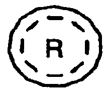 is selected from the group consisting of 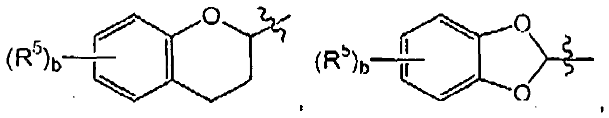 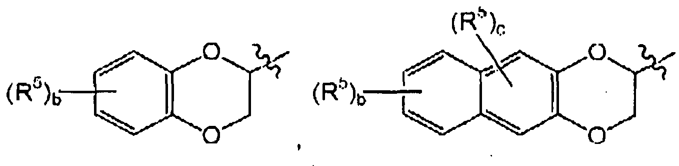 and 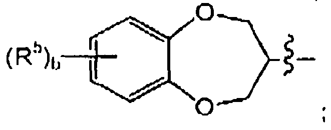 wherein b is an integer from 0 to 2; and wherein c is 0;
each R⁵ is independently selected from the group consisting of halogen, lower alkyl and nitro;
wherein "lower alkyl" refers to a carbon chain composition of 1-4 carbon atoms,
provided that when 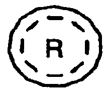 is 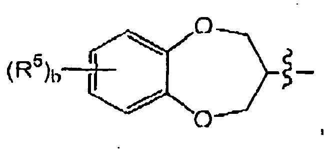 then a is 1.

7. The compound or a pharmaceutically acceptable salt thereof as in Claim 6, wherein
R¹ and R² are each independently selected from the group consisting of hydrogen and lower alkyl;
R⁴ is selected from the group consisting of hydrogen and methyl;
a is an integer from 1 to 2;
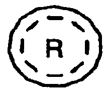 is selected from the group consisting of 2-(2,3-dihydro-benzo[1,4]dioxinyl), 2-(benzo[1,3]dioxolyl), 2-(3,4-dihydro-2H-benzo[1,4]dioxepinyl), 2-(2,3-dihydro-benzo[1,4]dioxinyl), 2-(6-chloro-2,3-dihydro-benzo[1,4]dioxinyl), 2-(6-fluoro-2,3-dihydro-benzo[1,4]dioxinyl), 2-(chromanyl), 2-(5-fluoro-2,3-dihydro-benzo[1,4]dioxinyl), 2-(7-chloro-2,3-dihydro-benzo[1,4]dioxinyl), 2-(6-chloro-benzo[1,3]dioxolyl), 2-(7-nitro-2,3-dihydro-benzo[1,4]dioxinyl), 2-(7-methyl-2,3-dihydro-benzo[1,4]dioxinyl), 2-(5-chloro-2,3-dihydro-benzo[1,4]dioxinyl), 2-(6-bromo-2,3-dihydro-benzo[1,4]dioxinyl), 2-(6,7-dichloro-2,3-dihydro-benzo[1,4]dioxinyl), 2-(8-chloro-2,3-dihydro-benzo[1,4]dioxinyl), 2-(2,3-dihydro-naphtho[2,3-b][1,4]dioxinyl) and 2-(4-methyl-benzo[1,3]dioxolyl);
wherein "lower alkyl" refers to a carbon chain composition of 1-4 carbon atoms,
provided that when 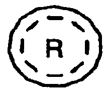 is 2-(3,4'-dihydro-2H-benzo[1,4]diaxepinyl), then a is 1.

8. The compound or a pharmaceutically acceptable salt thereof as in Claim 7, wherein
R¹ and R² are each independently selected from the group consisting of hydrogen and methyl;
R⁴ is selected from the group consisting of hydrogen and methyl;
a is an integer from 1 to 2;
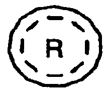 is selected from the group consisting of 2-(benzo[1,3]dioxolyl), 2-(2,3-dihydro-benzo[1,4]dioxinyl), 2-(2,3-dihydro-benzo[1,4]dioxinyl), 2-(6-chloro-2,3-dihydro-benzo[1,4]dioxinyl), 2-(7-chloro-2,3-dihydro-benzo[1,4]dioxinyl), 2-(7-methyl-2,3-dihydro-benzo[1,4]dioxinyl), 2-(6-bromo-2,3-dihydro-benzo[1,4]dioxinyl) and 2-(6,7-dichloro-2,3-dihydro-benzo[1,4]dioxinyl).

9. The compound of any of Claims 1 to 4, wherein the compound of formula (I) is selected from the group consisting of (2S)-(-)-N-(O-chloro-2,3-dihydro-benzo[1,4]dioxin-2-ylmethyl)-sulfamide; and pharmaceutically acceptable salts thereof.

10. A compound selected from the group consisting of (2S)-(-)-N-(6-chloro-2,3-dlhydro-benzo[1,4]dioxin-2-ylmethyl)-sulfamido; and pharmaceutically acceptable salts thereof for use in neuroprotection.

11. A compound selected from the group consisting of (2S)-(-)-N-(6-chloro-2,3-dihydro-benzo[1,4]dioxin-2-ylmethyl)-sulfamide; and pharmaceutically acceptable salts thereof for use in the treatment of an acute neurodegenerative disorder.

12. A compound selected from the group consisting of (2S)-(-)-N-(6-chloro-2,3-dihydro-benzo[1,4]dioxin-2-ylmethyl)-sulfamide; and pharmaceutically acceptable salts thereof for use in the treatment of a chronic neurodegenerative disorder.

13. A compound selected from the group consisting (2S)-(-)-N-(6-chloro-2,3-dihydro-benzo[1,4]dioxin-2-ylmethyl)-sulfamide; and pharmaceutically acceptable salts thereof for use in the prevention of neuron death or damage following brain, head or spinal cord trauma or injury.

14. A compound of formula (II) 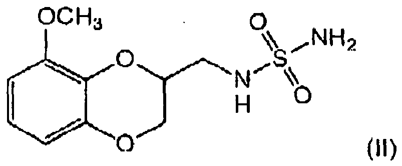 or a pharmaceutically acceptable salt thereof for use in neuroprotection.

15. A compound of formula (II) 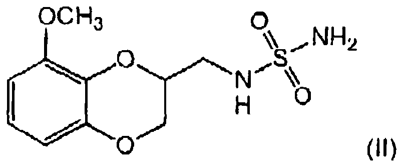 or a pharmaceutical acceptable salt thereof for use in the treatment of an acute neurodegenerative disorder.

16. A compound of formule (II) 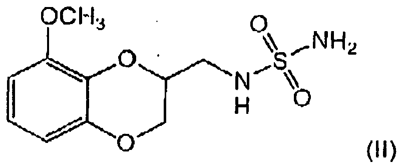 or a pharmaceutically acceptable salt thereof for use in the treatment of a chronic neurodegenerative disorder.

17. A compound of formula (II) 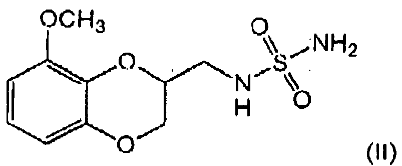 or a pharmaceutically acceptable salt thereof for use in the prevention of neuron death or damage following brain, head or spinal cord trauma or injury.

## Patentansprüche

1. Verbindung der Formel (I) 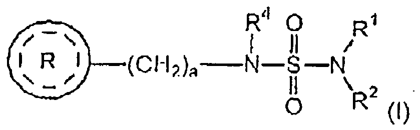 oder ein pharmazeutisch annehmbares Salz davon zur Verwendung bei der Neuroprotektion, wobei
R¹ und R² jeweils unabhängig voneinander aus der Gruppe bestehend aus Wasserstoff und Niederalkyl ausgewählt sind;
R⁴ aus der Gruppe bestehend aus Wasserstoff und Niederalkyl ausgewählt ist;
a für eine ganze Zahl von 1 bis 2 steht;
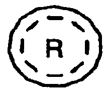 aus der Gruppe bestehend aus 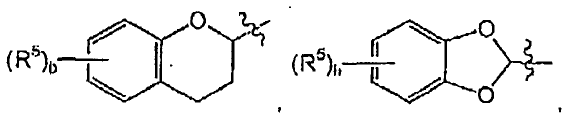 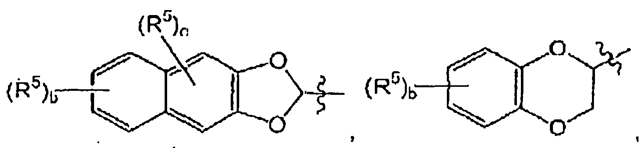 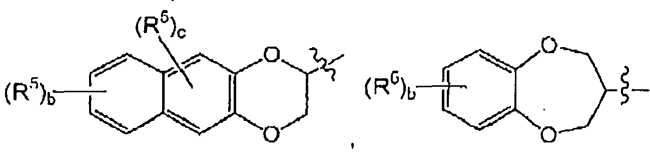 und 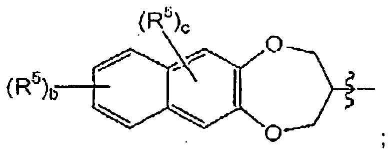 ausgewählt ist;
wobei b für eine ganze Zahl von 0 bis 4 steht und c für eine ganze Zahl von 0 bis 2 steht;
R⁵ jeweils unabhängig voneinander aus der Gruppe bestehend aus Halogen, Niederalkyl und Nitro ausgewählt ist;
wobei "Niederalkyl" sich auf eine Kohlenstoffkettenzusammensetzung mit 1-4 Kohlenstoffatomen bezieht,
mit der Maßqabe, daß dann, wenn 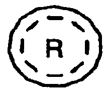 für 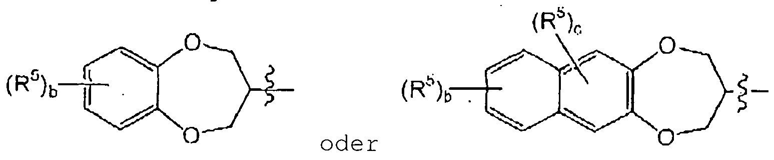 steht, a für 1 steht.

2. Verbindung der Formel (I) 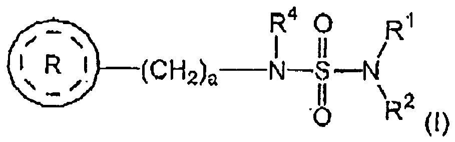 oder ein pharmazeutisch annehmbares Salz davon zur Verwendung bei der Behandlung einer akuten neurodegenerativen Störung,
wobei
R¹ und R² jeweils unabhängig voneinander aus der Gruppe bestehend aus Wasserstoff und Niederalkyl ausgewählt sind;
R⁴ aus der Gruppe bestehend aus Wasserstoff und Niederalkyl ausgewählt ist;
a für eine ganze Zahl von 1 bis 2 steht;
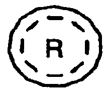 aus der Gruppe bestehend aus 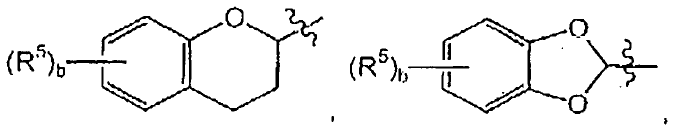 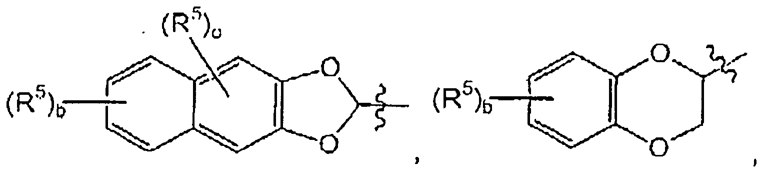 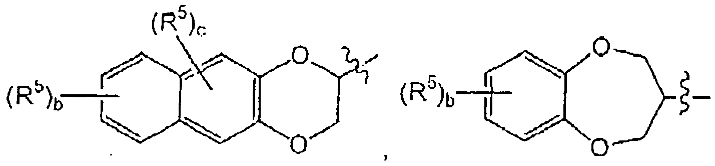 und 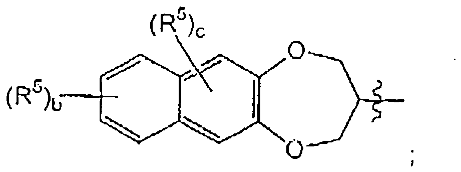 ausgewählt ist;
wobei b für eine ganze Zahl von 0 bis 4 steht und c für eine ganze Zahl von 0 bis 2 steht;
R⁵ jeweils unabhängig voneinander aus der Gruppe bestehend aus Halogen, Niederalkyl und Nitro ausgewählt ist;
wobei "Niederalkyl" sich auf eine Kohlenstoffkettenzusammensetzung mit 1-4 Kohlenstoffatomen bezieht,
mit der Maßgabe, daß dann, wenn 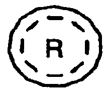 für 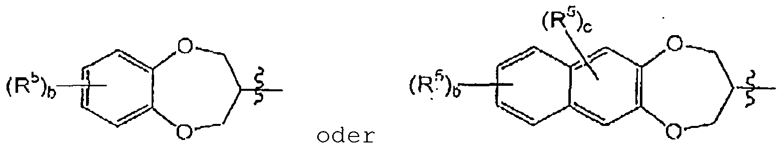 steht, a für 1 steht.

3. Verbindung der Formel (I) 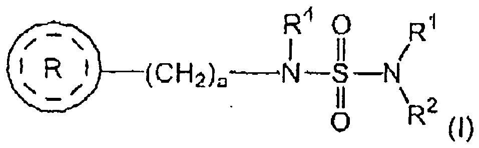 oder ein pharmazeutisch annehmbares Salz davon zur Verwendung bei der Behandlung einer chronischen neurodegenerativen Störung,
wobei
R¹ und R² jeweils unabhängig voneinander aus der Gruppe bestehend aus Wasserstoff und Niederalkyl ausgewählt sind;
R⁴ aus der Gruppe bestehend aus Wasserstoff und Niederalkyl ausgewählt ist;
a für eine ganze Zahl von 1 bis 2 steht;
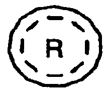 aus der Gruppe bestehend aus 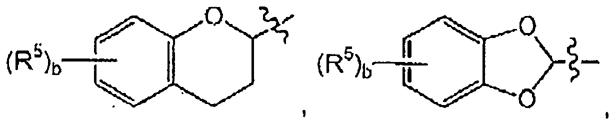 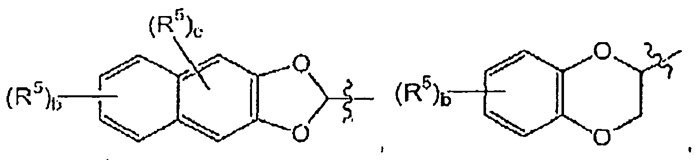 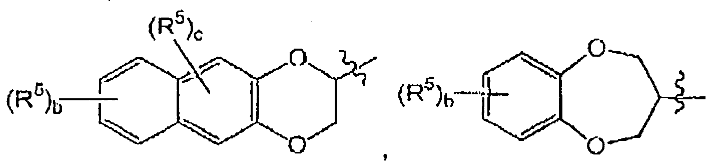 und 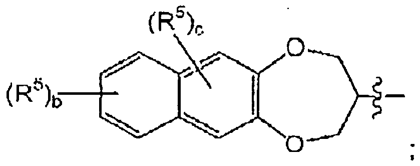 ausgewählt ist;
wobei b für eine ganze Zahl von 0 bis 4 steht und c für eine ganze Zahl von 0 bis 2 steht;
R⁵ jeweils unabhängig voneinander aus der Gruppe bestehend aus Halogen, Niederalkyl und Nitro ausgewählt ist;
wobei "Niederalkyl" sich auf eine Kohlenstoffkettenzusammensetzung mit 1-4 Kohlenstoffatomen bezieht,
mit der Maßgabe, daß dann, wenn 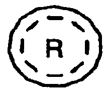 für 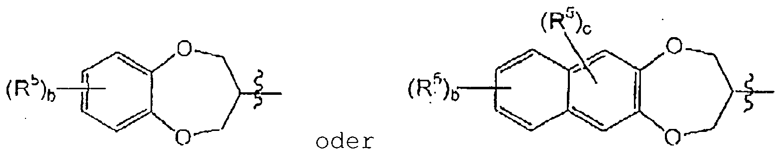 steht, a für 1 steht.

4. Verbindung der Formel (I) 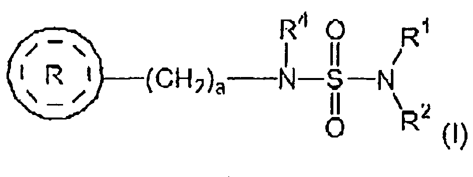 oder ein pharmazeutisch annehmbares Salz davon zur Verwendung bei der Prävention von Neuronentod oder -schaden nach Gehirn-, Kopf- oder Rückenmarkstrauma oder -verletzung,
wobei
R¹ und R² jeweils unabhängig voneinander aus der Gruppe bestehend aus Wasserstoff und Niederalkyl ausgewählt sind;
R⁴ aus der Gruppe bestehend aus Wasserstoff und Niederalkyl ausgewählt ist;
a für eine ganze Zahl von 1 bis 2 steht;
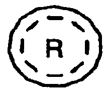 aus der Gruppe bestehend aus 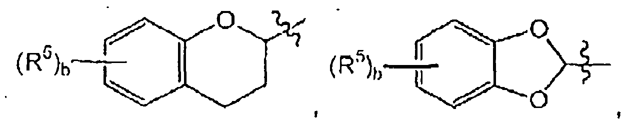 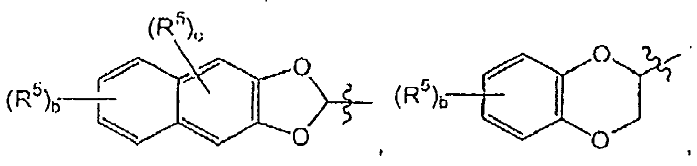 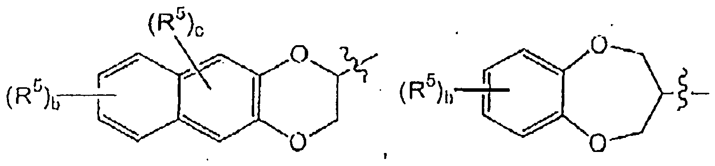 und 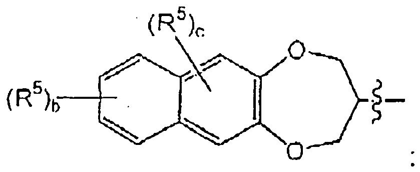 ausgewählt ist;
wobei b für eine ganze Zahl von 0 bis 4 steht und c für eine ganze Zahl von 0 bis 2 steht;
R⁵ jeweils unabhängig voneinander aus der Gruppe bestehend aus Halogen, Niederalkyl und Nitro ausgewählt ist;
wobei "Niederalkyl" sich auf eine Kohlenstoffkettenzusammensetzung mit 1-4 Kohlenstoffatomen bezieht,
mit der Maßgabe, daß dann, wenn 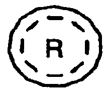 für 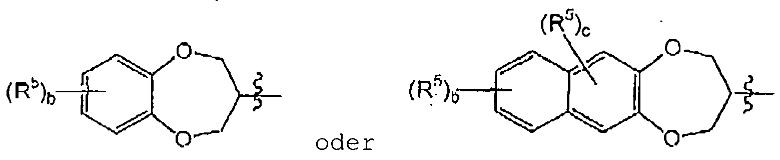 steht, a für 1 steht.

5. Verbindung oder pharmazeutisch annehmbares Salz davon nach einem der Ansprüche 1 bis 4, wobei R¹ und R² jeweils unabhängig voneinander aus der Gruppe bestehend aus Wasserstoff und Niederalkyl ausgewählt sind;
R⁴ aus der Gruppe bestehend aus Wasserstoff und Niederalkyl ausgewählt ist;
a für eine ganze Zahl von 1 bis 2 steht;
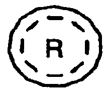 aus der Gruppe bestehend aus 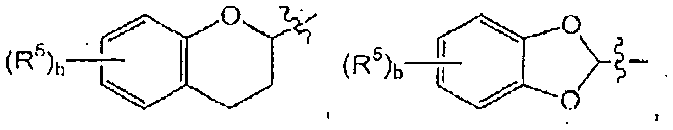 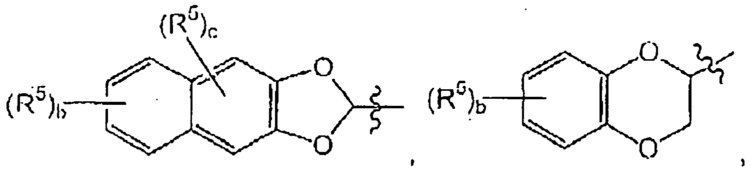 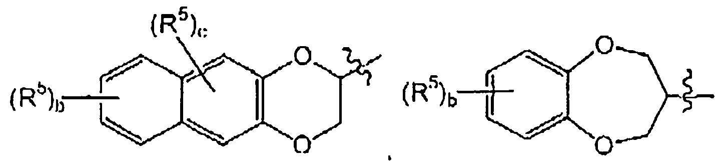 und 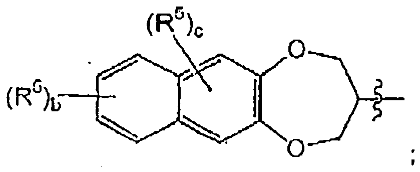 ausgewählt ist;
wobei b für eine ganze Zahl von 0 bis 2 steht und c für eine ganze Zahl von 0 bis 1 steht;
R⁵ jeweils unabhängig voneinander aus der Gruppe bestehend aus Halogen, Niederalkyl und Nitro ausgewählt ist;
wobei "Niederalkyl" sich auf eine Kohlenstoffkettenzusammensetzung mit 1-4 Kohlenstoffatomen bezieht,
mit der Maßgabe, daß dann, wenn 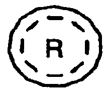 für 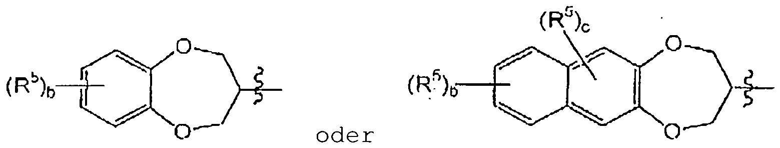 steht, a für 1 steht.

6. Verbindung oder pharmazeutisch annehmbares Salz davon nach Anspruch 5, wobei
R¹ und R² jeweils unabhängig voneinander aus der Gruppe bestehend aus Wasserstoff und Niederalkyl ausgewählt sind;
R⁴ aus der Gruppe bestehend aus Wasserstoff und Niederalkyl ausgewählt ist;
a für eine ganze Zahl von 1 bis 2 steht;
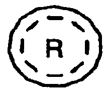 aus der Gruppe bestehend aus 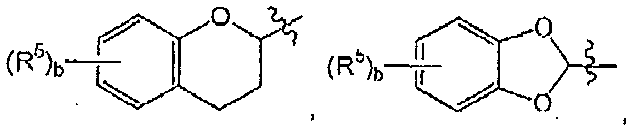 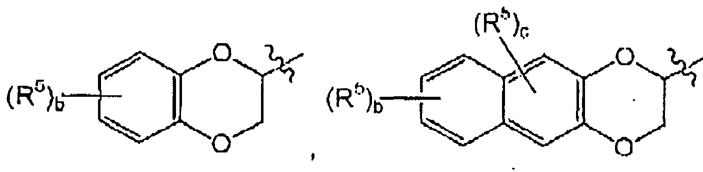 und 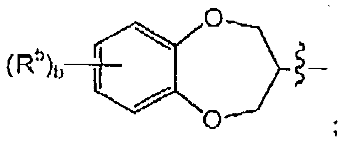 ausgewählt ist;
wobei b für eine ganze Zahl von 0 bis 2 steht und c für 0 steht;
R⁵ jeweils unabhängig voneinander aus der Gruppe bestehend aus Halogen, Niederalkyl und Nitro ausgewählt ist;
wobei "Niederalkyl" sich auf eine Kohlenstoffkettenzusammensetzung mit 1-4 Kohlenstoffatomen bezieht,
mit der Maßgabe, daß dann, wenn 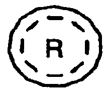 für 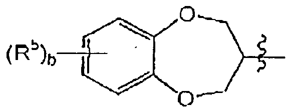 steht, a für 1 steht.

7. Verbindung oder pharmazeutisch annehmbares Salz davon nach Anspruch 6, wobei
R¹ und R² jeweils unabhängig voneinander aus der Gruppe bestehend aus Wasserstoff und Niederalkyl ausgewählt sind;
R⁴ aus der Gruppe bestehend aus Wasserstoff und Niederalkyl ausgewählt ist;
a für eine ganze Zahl von 1 bis 2 steht;
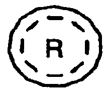 aus der Gruppe bestehend aus 2-(2,3-Dihydrobenzo[1,4]dioxinyl), 2-(Benzo[1,3]-dioxolyl), 2-(3,4-Dihydro-2H-benzo[1,4]dioxepinyl, 2-(2,3-Dihydrobenzo[1,4]dioxinyl, 2-(6-Chlor-2,3-dihydrobenzo[1,4]dioxinyl), 2-(6-Fluor-2,3-dihydrobenzo[1,4]dioxinyl), 2-(Chromanyl), 2-(5-Fluor-2,3-dihydrobenzo[1,4]dioxinyl), 2-(7-Chlor-2,3-dihydrobenzo[1,4]dioxinyl), 2-(6-Chlorbenzo-[1,3]dioxinyl, 2-(7-Nitro-2,3-dihydrobenzo[1,4]-dioxinyl), 2-(7-Methyl-2,3-dihydrobenzo[1,4]-dioxinyl), 2-(5-Chlor-2,3-dihydrobenzo[1,4]-dioxinyl), 2-(6-Brom-2,3-dihydrobenzo[1,4]-dioxinyl), 2-(6,7-Dichlor-2,3-dihydrobenzo[1,4]-dioxinyl), 2-(8-Chlor-2,3-dihydrobenzo[1,4]-dioxinyl), 2-(2,3-Dihydronaphtho[2,3-b][1,4]-dioxinyl) und 2-(4-Methylbenzo[1,3]dioxinyl) ausgewählt ist;
wobei "Niederalkyl" sich auf eine Kohlenstoffkettenzusammensetzung mit 1-4 Kohlenstoffatomen bezieht,
mit der Maßgabe, daß dann, wenn 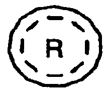 für 2-(3,4-Dihydro-2H-benzo[1,4]dioxepinyl) steht, a für 1 steht.

8. Verbindung oder pharmazeutisch annehmbares Salz davon nach Anspruch 7, wobei
R¹ und R² jeweils unabhängig voneinander aus der Gruppe bestehend aus Wasserstoff und Methyl ausgewählt sind;
R⁴ aus der Gruppe bestehend aus Wasserstoff und Methyl ausgewählt ist;
a für eine ganze Zahl von 1 bis 2 steht;
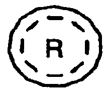 aus der Gruppe bestehend aus 2-(Benzo[1,3]-dioxolyl), 2-(2,3-Dihydrobenzo[1,4]dioxinyl, 2-(2,3-Dihydrobenzo[1,4]dioxinyl, 2-(6-Chlor-2,3-dihydrobenzo[1,4]dioxinyl), 2-(7-Chlor-2,3-dihydrobenzo[1,4]dioxinyl), 2-(7-Methyl-2,3-dihydrobenzo-[1,4]dioxinyl), 2-(6-Brom-2,3-dihydrobenzo[1,4]-dioxinyl) und 2-(6,7-Dichlor-2,3-dihydrobenzo-[1,4]dioxinyl) ausgewählt ist.

9. Verbindung nach einem der Ansprüche 1 bis 4, wobei die Verbindung der Formel (I) aus der Gruppe bestehend aus (2S) - (-) -N- (6-Chlor-2,3-dihydrobenzo[1,4]dioxin-2-ylmethyl)sulfamid und pharmazeutisch annehmbaren Salzen davon ausgewählt ist.

10. Verbindung aus der Gruppe bestehend aus (2S)-(-)-N-(6-Chlor-2,3-dihydrobenzo[1,4]dioxin-2-ylmethyl)sulfamid und pharmazeutisch annehmbaren Salzen davon zur Verwendung bei der Neuroprotektion.

11. Verbindung aus der Gruppe bestehend aus (2S)-(-)-N-(6-Chlor-2,3-dihydrobenzo[1,4]dioxin-2-ylmethyl)sulfamid und pharmazeutisch annehmbaren Salzen davon zur Verwendung bei der Behandlung einer akuten neurodegenerativen Störung.

12. Verbindung aus der Gruppe bestehend aus (2S)-(-)-N-(6-Chlor-2,3-dihydrobenzo[1,4]dioxin-2-ylmethyl)sulfamid und pharmazeutisch annehmbaren Salzen davon zur Verwendung bei der Behandlung einer chronischen neurodegenerativen Störung.

13. Verbindung aus der Gruppe bestehend aus (2S)-(-)-N-(6-Chlor-2,3-dihydrobenzo[1,4]dioxin-2-ylmethyl)sulfamid und pharmazeutisch annehmbaren Salzen davon zur Verwendung bei der Prävention von Neuronentod oder -schaden nach Gehirn-, Kopf- oder Rückenmarkstrauma oder -verletzung.

14. Verbindung der Formel (II) 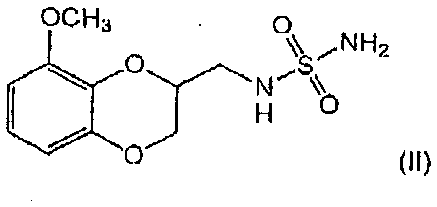 oder ein pharmazeutisch annehmbares Salz davon zur Verwendung bei der Neuroprotektion.

15. Verbindung der Formel (II) 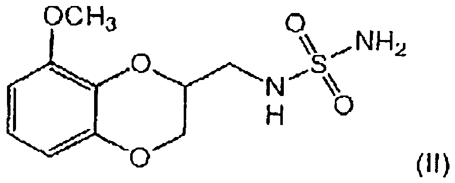 oder ein pharmazeutisch annehmbares Salz davon zur Verwendung bei der Behandlung einer akuten neurodegenerativen Störung.

16. Verbindung der Formel (II) 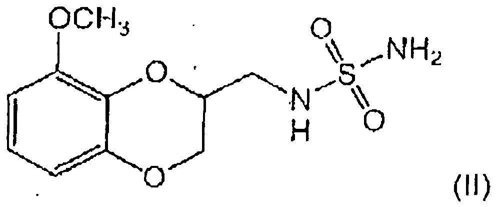 oder ein pharmazeutisch annehmbares Salz davon zur Verwendung bei der Behandlung einer chronischen neurodegenerativen Störung.

17. Verbindung der Formel (II) 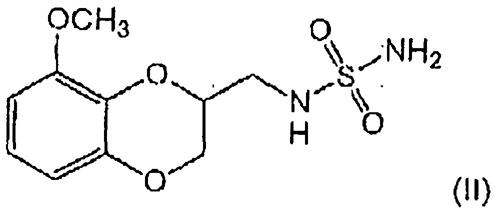 oder ein pharmazeutisch annehmbares Salz davon zur Verwendung bei der Prävention von Neuronentod oder -schaden nach Gehirn-, Kopf- oder Rückenmarkstrauma oder -verletzung.

## Revendications

1. Composé de formule (I) : 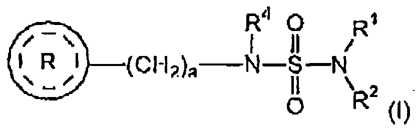 ou l'un de ses sels de qualité pharmaceutique pour emploi en neuroprotection, où
chacun des radicaux R¹ et R² est indépendamment choisi dans le groupe constitué par l'atome d'hydrogène et les groupements alkyle courts ;
R⁴ est choisi dans le groupe constitué par l'atome d'hydrogène et les groupements alkyle courts ;
a représente un entier compris entre 1 et 2 ;
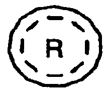 est choisi dans le groupe constitué par les groupements suivantes : 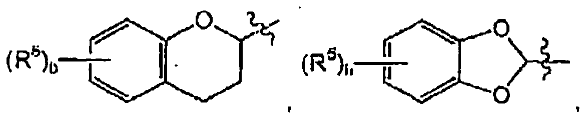 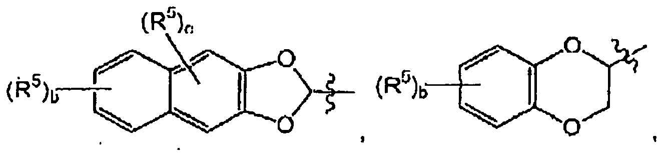 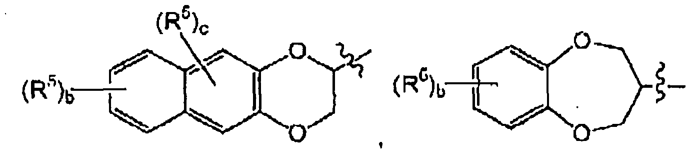 et 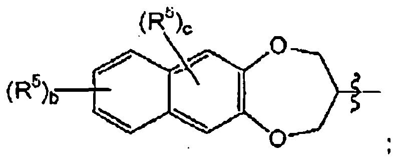 où b représente un entier compris entre 0 et 4 ; et où c représente un entier compris entre 0 et 2 ;
chacun des radicaux R⁵ est indépendamment choisi dans le groupe constitué par les atomes d'halogène et les groupements alkyle courts et nitro ;
le terme « alkyle court » désignant une chaîne carbonée composée de 1 à 4 atomes de carbone,
à la condition que lorsque 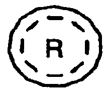 représente 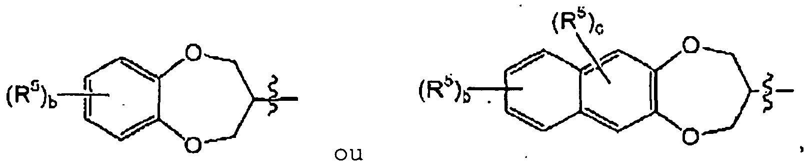 a est égal à 1.

2. Composé de formule (I) : 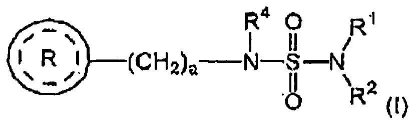 ou l'un de ses sels de qualité pharmaceutique, pour emploi dans le traitement d'un trouble neurodégénératif aigu,
où
chacun des radicaux R¹ et R² est indépendamment choisi dans le groupe constitué par l'atome d'hydrogène et les groupements alkyle courts ;
R⁴ est choisi dans le groupe constitué par l'atome d'hydrogène et les groupements alkyle courts ;
a représente un entier compris entre 1 et 2 ;
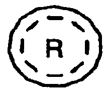 est choisi dans le groupe constitué par les groupements suivantes : 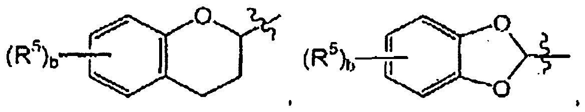 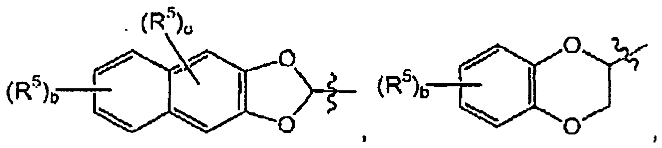 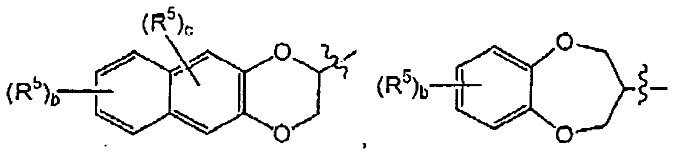 et 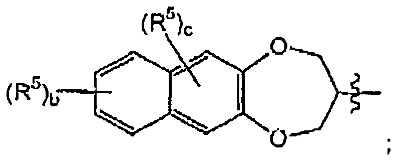 où b représente un entier compris entre 0 et 4 ; et où c représente un entier compris entre 0 et 2 ;
chacun des radicaux R⁵ est indépendamment choisi dans le groupe constitué par les atomes d'halogène et les groupements alkyle courts et nitro ;
le terme « alkyle court » désignant une chaîne carbonée composée de 1 à 4 atomes de carbone,
à la condition que lorsque 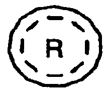 représente 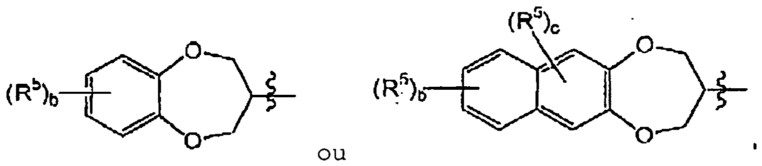 a est égal à 1.

3. Composé de formule (I) : 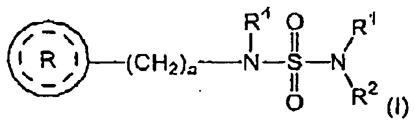 ou l'un de ses sels de qualité pharmaceutique, pour emploi dans le traitement d'un trouble neurodégénératif chronique,
où
chacun des radicaux R¹ et R² est indépendamment choisi dans le groupe constitué par l'atome d'hydrogène et les groupements alkyle courts ;
R⁴ est choisi dans le groupe constitué par l'atome d'hydrogène et les groupements alkyle courts ;
a représente un entier compris entre 1 et 2 ;
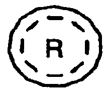 est choisi dans le groupe constitué par les groupements suivantes : 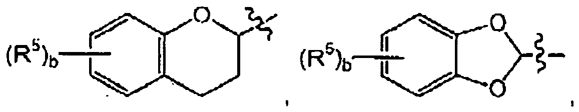 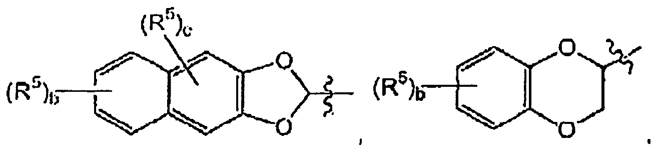 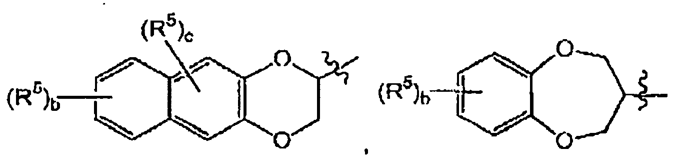 et 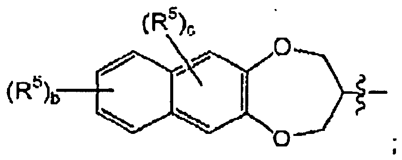 où b représente un entier compris entre 0 et 4 ; et où c représente un entier compris entre 0 et 2 ;
chacun des radicaux R⁵ est indépendamment choisi dans le groupe constitué par les atomes d'halogène et les groupements alkyle courts et nitro ;
le terme « alkyle court » désignant une chaîne carbonée composée de 1 à 4 atomes de carbone,
à la condition que lorsque 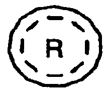 représente 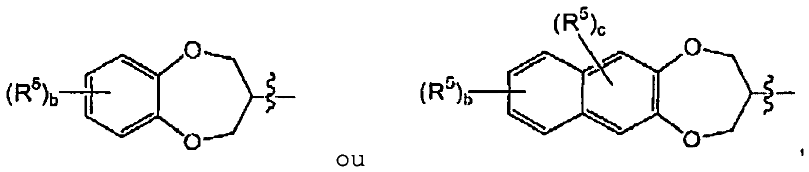 a est égal à 1.

4. Composé de formule (I) : 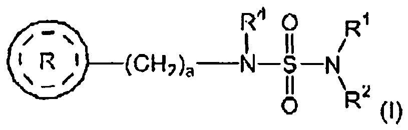 ou l'un de ses sels de qualité pharmaceutique, pour emploi dans la prévention de la mort neuronale ou des dommages suivant un traumatisme ou une lésion au cerveau, à la tête ou à la moelle épinière,
où
chacun des radicaux R¹ et R² est indépendamment choisi dans le groupe constitué par l'atome d'hydrogène et les groupements alkyle courts ;
R⁴ est choisi dans le groupe constitué par l'atome d'hydrogène et les groupements alkyle courts ;
a représente un entier compris entre 1 et 2 ;
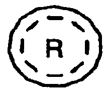 est choisi dans le groupe constitué par les groupements suivants : 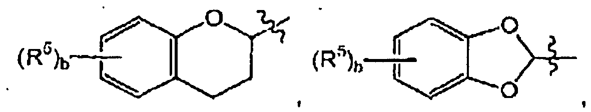 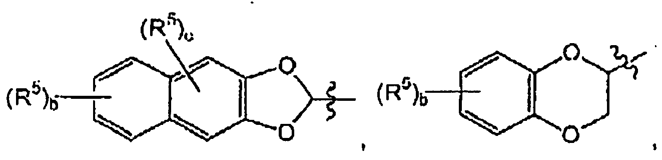 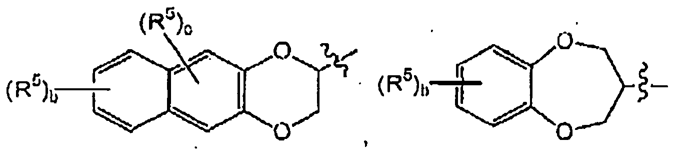 et 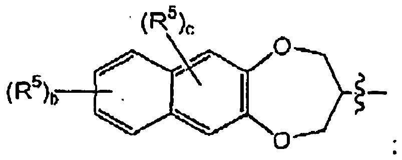 où b représente un entier compris entre 0 et 4 ; et où c représente un entier compris entre 0 et 2 ;
chacun des radicaux R⁵ est indépendamment choisi dans le groupe constitué par les atomes d'halogène et les groupements alkyle courts et nitro ;
le terme « alkyle court » désignant une chaîne carbonée composée de 1 à 4 atomes de carbone,
à la condition que lorsque 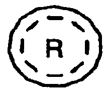 représente 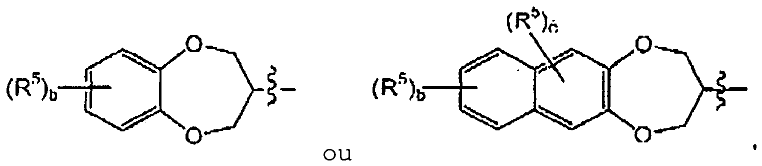 a est égal à 1.

5. Composé ou l'un de ses sels de qualité pharmaceutique conforme à l'une quelconque des revendications 1 à 4, où
chacun des radicaux R¹ et R² est indépendamment choisi dans le groupe constitué par l'atome d'hydrogène et les groupements alkyle courts ;
R⁴ est choisi dans le groupe constitué par l'atome d'hydrogène et les groupements alkyle courts ;
a représente un entier compris entre 1 et 2 ;
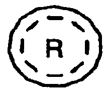 est choisi dans le groupe constitué par les groupements suivants : 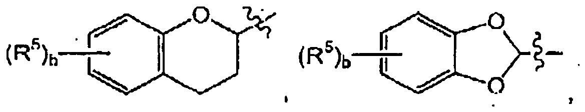 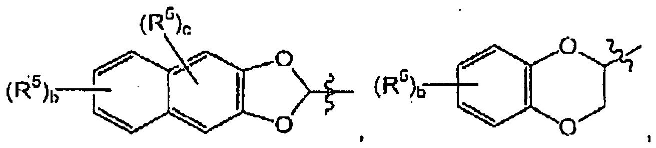 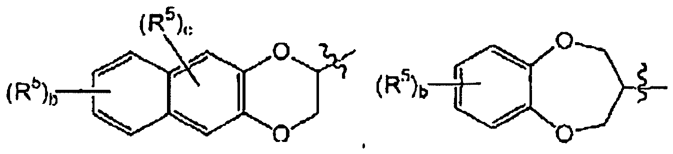 et 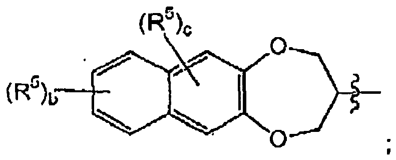 où b représente un entier compris entre 0 et 2 ; et où c représente un entier compris entre 0 et 1 ;
chacun des radicaux R⁵ est indépendamment choisi dans le groupe constitué par les atomes d'halogène et les groupements alkyle courts et nitro ;
le terme « alkyle court » désignant une chaîne carbonée composée de 1 à 4 atomes de carbone,
à la condition que lorsque 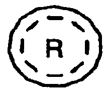 représente 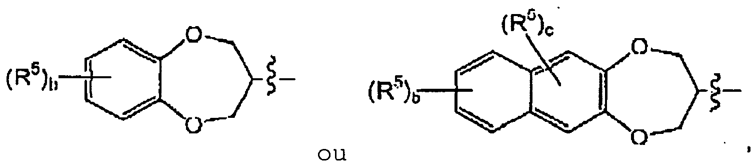 a est égal à 1.

6. Composé ou l'un de ses sels de qualité pharmaceutique conforme à la revendication 5, où
chacun des radicaux R¹ et R² est indépendamment choisi dans le groupe constitué par l'atome d'hydrogène et les groupements alkyle courts ;
R⁴ est choisi dans le groupe constitué par l'atome d'hydrogène et les groupements alkyle courts ;
a représente un entier compris entre 1 et 2 ;
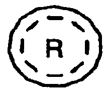 est choisi dans le groupe constitué par les groupements suivants : 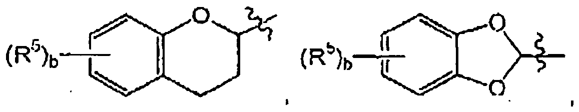 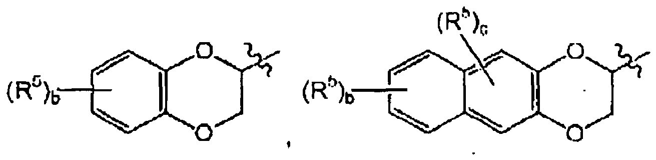 et 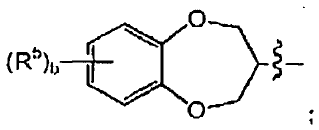 où b représente un entier compris entre 0 et 2 ; et où c est égal à 0 ;
chacun des radicaux R⁵ est indépendamment choisi dans le groupe constitué par les atomes d'halogène et les groupements alkyle courts et nitro ;
le terme « alkyle court » désignant une chaîne carbonée composée de 1 à 4 atomes de carbone,
à la condition que lorsque 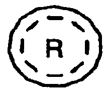 représente 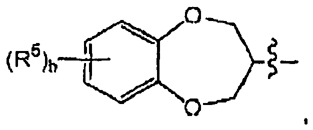 a est égal à 1.

7. Composé ou l'un de ses sels de qualité pharmaceutique conforme à la revendication 6, où chacun des radicaux R¹ et R² est indépendamment choisi dans le groupe constitué par l'atome d'hydrogène et les groupements alkyle courts ;
R⁴ est choisi dans le groupe constitué par l'atome d'hydrogène et le groupement méthyle ;
a représente un entier compris entre 1 et 2 ;
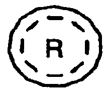 est choisi dans le groupe constitué par les groupements suivants : 2-(2,3-dihydro-benzo[1,4]dioxinyle), 2-(benzo[1,3]dioxolyle), 2-(3,4-dihydro-2H-benzo[1,4]dioxépinyle), 2-(2,3-dihydro-benzo[1,4]dioxinyle), 2-(6-chloro-2,3-dihydro-benzo[1,4]dioxinyle), 2-(6-fluoro-2,3-dihydro-benzo[1,4]dioxinyle), 2-(chromanyle), 2-(5-fluoro-2,3-dihydro-benzo[1,4]dioxinyle), 2-(7-chloro-2,3-dihydro-benzo[1,4]dioxinyle), 2-(6-chloro-benzo[1,3]dioxolyle), 2-(7-nitro-2,3-dihydro-benzo[1,4]dioxinyle), 2-(7-méthyl-2,3-dihydro-benzo[1,4]dioxinyle), 2-(5-chloro-2,3-dihydro-benzo[1,4]dioxinyle), 2-(6-bromo-2,3-dihydro-benzo[1,4]dioxinyle), 2-(6,7-dichloro-2,3-dihydro-benzo[1,4]dioxinyle), 2-(8-chloro-2,3-dihydro-benzo[1,4]dioxinyle), 2-(2,3-dihydro-naphto[2,3-b][1,4]dioxinyle) et 2-(4-méthyl-benzo[1,3]dioxolyle) ; le terme « alkyle court » désignant une chaîne carbonée composée de 1 à 4 atomes de carbone,
à la condition que lorsque 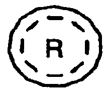 représente un groupement 2-(3,4-dihydro-2H-benzo[1,4]dioxépinyle), a est égal à 1.

8. Composé ou l'un de ses sels de qualité pharmaceutique conforme à la revendication 7, où
chacun des radicaux R¹ et R² est indépendamment choisi dans le groupe constitué par l'atome d'hydrogène et le groupement méthyle ;
R⁴ est choisi dans le groupe constitué par l'atome d'hydrogène et le groupement méthyle ;
a représente un entier compris entre 1 et 2 ;
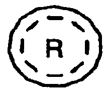 est choisi dans le groupe constitué par les groupements suivants : 2-(benzo[1,3]dioxolyle), 2-(2,3-dihydro-benzo[1,4]dioxinyle), 2-(2,3-dihydro-benzo[1,4]dioxinyle), 2-(6-chloro-2,3-dihydro-benzo[1,4]dioxinyle), 2-(7-chloro-2,3-dihydro-benzo[1,4]dioxinyle), 2-(7-méthyl-2,3-dihydro-benzo[1,4]dioxinyle), 2-(6-bromo-2,3-dihydro-benzo[1,4]dioxinyle) et 2-(6,7-dichloro-2,3-dihydro-benzo[1,4]dioxinyle).

9. Composé conforme à l'une quelconque des revendications 1 à 4, où le composé de formule (I) est choisi dans le groupe constitué par le (2S)-(-)-N-(6-chloro-2,3-dihydro-benzo[1,4]dioxin-2-ylméthyl)-sulfamide et ses sels de qualité pharmaceutique.

10. Composé choisi dans le groupe constitué par le (2S)-(-)-N-(6-chloro-2,3-dihydro-benzo[1,4]dioxin-2-ylméthyl)-sulfamide et ses sels de qualité pharmaceutique pour emploi en neuroprotection.

11. Composé choisi dans le groupe constitué par le (2S)-(-)-N-(6-chloro-2,3-dihydro-benzo[1,4]dioxin-2-ylméthyl)-sulfamide et ses sels de qualité pharmaceutique pour emploi dans le traitement d'un trouble neurodégénératif aigu.

12. Composé choisi dans le groupe constitué par le (2S)-(-)-N-(6-chloro-2,3-dihydro-benzo[1,4]dioxin-2-ylméthyl)-sulfamide et ses sels de qualité pharmaceutique pour emploi dans le traitement d'un trouble neurodégénératif chronique.

13. Composé choisi dans le groupe constitué par le (2S)-(-)-N-(6-chloro-2,3-dihydro-benzo[1,4]dioxin-2-ylméthyl)-sulfamide et ses sels de qualité pharmaceutique, pour emploi dans la prévention de la mort neuronale ou des dommages consécutifs à un traumatisme ou une lésion du cerveau, de la tête ou de la moelle épinière.

14. Composé de formule (II) : 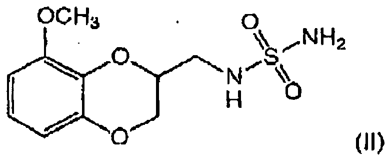 ou l'un de ses sels de qualité pharmaceutique pour emploi en neuroprotection.

15. Composé de formule (II) : 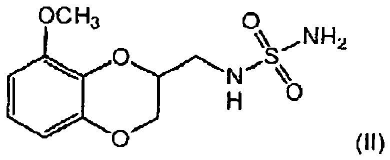 ou l'un de ses sels de qualité pharmaceutique, pour emploi dans le traitement d'un trouble neurodégénératif aigu.

16. Composé de formule (II) : 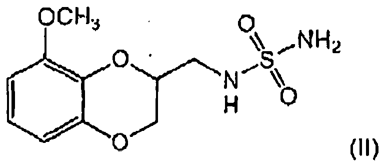 ou l'un de ses sels de qualité pharmaceutique, pour emploi dans le traitement d'un trouble neurodégénératif chronique.

17. Composé de formule (II) : 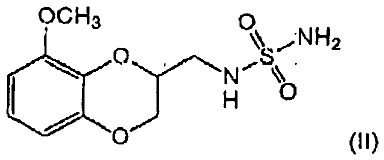 ou l'un de ses sels de qualité pharmaceutique, pour emploi dans la prévention de la mort neuronale ou des dommages suivant un traumatisme ou une lésion au cerveau, à la tête ou à la moelle épinière.
